# EUROPEAN PATENT APPLICATION

(11) **EP 1 797 901 A1**
(43) Date of publication of application: **20.06.2007**
(21) Application number: 05292722.5
(22) Date of filing: 16.12.2005
(51) Int. Cl.: A61K 47/48, A61K 31/74, C08G 65/329

(54) **Cell penetrating peptide conjugates for delivering nucleic acids into cells**

(71) Applicant: Diatos, 75014 Paris (FR)
(72) Inventor: Alluis, Bertrand, F-69100 Villeurbanne (FR); Fruchart, Jean-Sébastien, F-78390 Bois d'Arcy (FR)
(74) Representative: Breese Derambure Majerowicz

(57) **Abstract**

The invention provides cell penetrating peptide-nucleic acid conjugates having the formula P-L-N, wherein P is a cell penetrating peptide, N is a nucleic acid, preferably an oligonucleotide and more preferably a siRNA, and L is a hydrophilic polymer, preferably a polyethylene glycol (PEG)-based linker linking P and N together. Compositions, methods of use and methods for producing such conjugates are also disclosed.

## Description

The invention relates generally to delivery of nucleic acids using cell penetrating peptides. More particularly it concerns cell penetrating peptide-nucleic acid conjugates enabling an efficient delivery of said nucleic acid into cells both *in vitro* and *in vivo.*

Genetic information now available from the human genome sequence may be exploited for the design of specific agents to modulate the function of genes or their protein products to correct genetic disorders or to treat diseases, such as cancers. A number of strategies have been developed to manipulate gene expression: gene therapies, ribozymes, antisense RNAs or small interfering RNAs (siRNA shRNA or RNAi). However, these strategies are hampered by the relatively inefficient delivery of genetic material into either somatic or cultured cells. Methods for delivering nucleic acids into cells include microinjection, electroporation, particle bombardment, transfection using viral systems such as retrovirus and adenovirus, or non-viral systems such as cationic liposomes, cationic lipids, cholesterol or polymers, dendrimers or cell penetrating peptides (Lochmann et al., Eur. J. Pharmaceut. Biopharmaceut. 58:237-251 (2004)). These methods all have limitations generally with regard to: efficiency of delivery, low concentration of nucleic acid delivered into the target cell or organ to give a biological effect *in vivo,* potential size-limit of the nucleic acid being transported, safety concerns and/or production scale-up difficulties. Thus, there is considerable interest in the further development of delivery systems for nucleic acids.

RNA interference (RNAi) is a natural process whereby double-stranded RNA (dsRNA) induces the sequence-specific degradation of homologous messenger RNA (mRNA). Small interfering RNAs (siRNAs) constitute a powerful tool to silence gene expression posttranscriptionally (Hannon, Nature 418:244-251 (2002); McManus and Sharp, Nat. Rev. Genet. 3:737-747 (2002); Elbashir et al., Nature 411:494-498 (2001); Agami, Curr. Opin. in Chem. Biol., 6:829-834(2002)). RNAi is an ATP dependant, processive cleavage of the double stranded RNA into 21-23 nucleotide siRNAs by the enzyme called RNase III Dicer. These siRNAs are incorporated into various protein factors and form RNA induced silencing complex (RISC) (Hammond et al., Nature 404:293-296 (2000)). ATP-dependant unwinding of the siRNA duplex generates an active complex, RISC* (the asterisk indicates an active conformation of the complex). Guided by the antisense strand of siRNA, RISC* recognizes and cleaves the complementary mRNA in the cytoplasm with the help of endoribonucleases.

The major limitation of siRNA application, as for antisense RNA or nucleic acid-based strategies, remains their poor cellular uptake related to low permeability of the cell membrane to nucleic acids (Luo and Saltzman, Nat. Biotechnol. 18:33-37 (2000); Niidome and Huang, Gene Ther. 10:991-998 (2002)). Although siRNA transfection can be achieved in classical laboratory cultured cell lines using lipid-based formulations, siRNA delivery remains a major challenge for many cell lines and a need for improving efficient method for *in vivo* application still exists (Rozema and Lewis, Target 2:253-260 (2003)).

Several peptide-based strategies have been developed to improve the delivery of oligonucleotides both *in vitro* and *in vivo* using covalent or mixing (complex) approaches (Morris et al., Curr. Opin. Biotechnol. 11:461-466 (2000); Järver and Langel, Drug Discovery Today 9:395-402 (2004); Gait, Cell. Mol. Life Sci. 60:1-10 (2003)). Among these strategies, the coupling of nucleic acids to certain peptides can enhance intracellular delivery. Indeed, cell penetrating peptides (CPPs) are useful carrier for cellular uptake of oligonucleotides (Juliano, Curr. Opin. Mol. Ther. 7:132-136 (2005)). However, there is currently substantial interest in the synthesis and biological properties of CPP-nucleic acid conjugates with enhanced delivery efficiency.

Within the framework of research that has led to this invention, the applicant prepared cell penetrating peptide-small interfering RNA conjugates by using different linker groups between the CPP and the siRNA. Then, siRNA conjugated to CPPs were evaluated *in vitro* and *in vivo* for siRNA uptake efficiency and resulting siRNA activity.

There are numerous methods reported for synthesis of peptide-oligonucleotide conjugates; *e.g*., reviewed in Zubin et al. (Russ. Chem. Rev. 71:239-264 (2002)). However, in many cases, when one tries to conjugate basic amino acid-rich peptides to a oligonucleotide in solution-phase, the reaction can not be carried out because of serious precipitation caused by electrostatic interaction of the two moieties.

Chiu et al. (Chemistry & Biology, 11:1165-1175 (2004)) have reported that functional siRNA can be delivered into cells by using siRNA conjugated to the 11 amino acid cationic peptide sequence that corresponds to amino acid 47-57 within HIV-1 Tat protein. siRNA-TAT peptide conjugates were created by annealing 21 nucleotide 5' Cy3-labeled sense strand siRNA to 3'-N3 modified antisense strand siRNA. Duplex siRNA with 3'-N3 modification were then incubated with a heterobifunctional crosslinker, sulfosuccinimidyl 4-(p-maleimidophenyl)-butyrate. During this step, the NHS ester group of the crosslinker reacted with the primary amino group at the 3' termini of the siRNA. A cysteine residue added to the amino termini of the TAT(47-57) peptide or TAT(47-57)-derived oligocarbamate was used for siRNA conjugation, during which the maleimido group of the crosslinker reacted with the sulfhydryl group of the cysteine residue of the peptide.

Muratovska and Eccles (FEBS Letters 558:63-68 (2004) and Erratum in FEBS Lett. 566:317 (2004)) described conjugates for directly targeting siRNA to the cytoplasm of cells, with delivery across the plasma membrane using the cell penetrating peptides, penetratin and transportan. The authors reported the synthesis of disulfide-linked CPP-siRNA.

Moulton et al. (Bioconjug Chem. 15:290-9 (2004)) have assessed the cellular uptake of antisense morpholino oligomers conjugated to arginine-rich peptides. The linker effects on intracellular distribution and concentration of the peptide conjugated to phosphorodiamidate morpholino oligomers (PMO) were also evaluated with the following bifunctional crosslinkers: N-[epsilon-maleimidocaproyloxy] sulfosuccinimide ester (sulfo-EMCS), *N*-[gamma-maleimidobutyryloxy] succinimide ester (GMBS), *N* [-kappa-maleimidoundecanoyloxy) sulfosuccinimide ester (KMUS), succinimidyl 3-[bromoacetamido] propionate (SBAP), *N-*succinimidyl 3-[2-pyridyldithio] propionate (SPDP) and sulfosuccinimidyl 6-[3'-(2-pyridyldithio) propionamido]hexanoate (sulfo-LCSPDP). It was shown that the antisense activity of the PMO was increased when conjugated to a peptide with longer linkers. Then, it was presumed that the higher antisense activity of the conjugates with longer chains of (CH₂)ₙ moieties may be the result of their greater flexibility and/or hydrophobicity than those with shorter linkers. Further, results indicate that the delivery peptide had greater influence on subcellular distribution of PMO with non-cleavable linker (thiomaleimide linker) compared to cleavable linker (disulfide linker).

U.S. published application 2004/0147027 discloses a complex comprising double-stranded ribonucleic acid molecule, a cell penetrating peptide, and a covalent bond linking the double-stranded ribonucleic acid molecule to the cell penetrating peptide. The bond linking the cell penetrating peptide and the modified strand of the ribonucleic acid molecule can be a disulfide bond, ester bond, carbamate bond or sulfonate bond.

PCT patent application WO 04/048545 describes siRNAs crosslinked with TAT peptide 47-57 using sulfosuccinimidy14-[p-maleimidophenyl] butyrate crosslinkers (Sulfo-SMPB).

Zanta et al. (Proc Natl Acad Sci USA 96:91-6 (1999)) linked a single nuclear localization signal peptide to a capped CMVLuciferase oligonucleotide using *N*-succinimidyl-4-(*N-*maleimidomethyl)-cyclohexane-1-carboxylate (SMCC) as a linker and transfected cells *in vitro.*

PCT patent application WO 99/05302 discloses nucleic acid analogs (*e.g*., an antisense molecule) coupled to a transport peptide (such as transportan or penetratin) which effects transport across a lipid membrane and then permits presentation of the nucleic acid analog to intracellular polynucleotides, whereby duplex or triplex structures are formed. The hybridizing nucleic acid analog and the transport peptide are attached by a bond which is labile in the intracellular environment so that the hybridizing nucleic acid analog is cleaved and released, thereby physically separating the nucleic acid analog and peptide moieties. An example of a labile bond is a disulfide bond whereupon exposure to an endogenous intracellular enzyme or reducing agent, such as glutathione or NADPH, the disulfide bond is broken or cleaved, separating the peptide and nucleic acid analogs moieties.

The present invention is based on the discovery that nucleic acid molecules such as oligonucleotides or specifically siRNA molecules can be conjugated to cell penetrating peptides with a hydrophilic polymer, preferably a polyethylene glycol-based linker such that properties important for *in vivo* applications, in particular, human therapeutic applications, are improved without compromising the nucleic acid, oligonucleotide or siRNA molecule activity.

This invention specifically has as its object to offer new cell penetrating peptide-nucleic acid conjugates with improved delivery efficacy into cells both *in vitro* and *in vivo.*

This object is attained by using a hydrophilic polymer, preferably a polyethylene glycol (PEG)-based linker for conjugating the cell penetrating peptide and the nucleic acid.

In accordance with the present invention, there is provided a cell penetrating peptide-nucleic acid ("CPP-nucleic acid") conjugate having the formula I: P-L-N, wherein P is a cell penetrating peptide, N is a nucleic acid, preferably an oligonucleotide and more preferably a siRNA, and L is a hydrophilic polymer, preferably a polyethylene glycol (PEG)-based linker linking P and N together.

The present invent has as object a compound having the formula I: 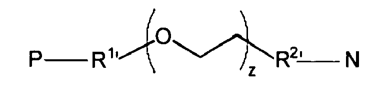 wherein
P is a cell penetrating peptide,
N is a nucleic acid,
R^{1'} and R^{2'} are divalent organic radicals independently selected from substituted or unsubstituted alkyl, substituted or unsubstituted heteroalkyl and substituted or unsubstituted aryl;
z is an integer from 1 to 10000.

Accordingly, the present invent has as object a compound having the formula P-L-N, wherein L represents a PEG based linker, which can be obtained by the conjugation of P and N together with a group of formula II 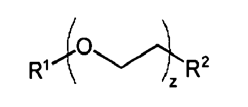 wherein
R¹ and R² are independently selected from =O, SR³, -NHR³ and -OR³, in which R³ is H, substituted or unsubstituted alkyl, substituted or unsubstituted heteroalkyl, acyl, -OR⁴, -C(O)R⁴, -C(O)OR⁴, -C(O)NR⁴R⁵, -P(O)(OR⁴)₂, -C(O)CHR⁴R⁵, -NR⁴R⁵, -N(+)R⁴R⁵R⁶, -SR⁴ or SiR⁴R⁵R⁶; the symbols R⁴, R⁵ and R⁶ independently represent H, substituted or unsubstituted alkyl, substituted or unsubstituted heteroalkyl and substituted or unsubstituted aryl, wherein R⁴ and R⁵ together with the nitrogen atom to which they are attached are optionally joined to form a substituted or unsubstituted heterocycloalkyl ring system having from 4 to 6 members, optionally containing two or more heteroatoms;
z is an integer from 1 to 10000; and
wherein R¹ has reacted with P and R² has reacted with N.

The terms "conjugate" or "conjugated" refer to a covalent, ionic, or hydrophobic interaction whereby the moieties of a molecule are held together and preserved in proximity.

The term "reacted" has the ordinary meaning for one skilled in the art of chemistry.

The terms "linker" or "crosslinker" are used interchangeably herein and refer to one or more atoms comprising a chain conjugating a nucleic acid or nucleic acid analog to a moiety such as a cell penetrating peptide, label, modifier stabilizing group, or the like.

The term *"in vitro"* has its art recognized meaning, e.g., cell culture, involving purified reagents or extracts, e.g., cell extracts.

The term *"in vivo"* also has its art recognized meaning, *e.g.,* involving immortalized cells, primary cells, cell lines, and/or cells in an organism.

The term "peptide(s)" refer to a polymer of amino acids of which the written convention is N, or amino, terminus is on the left and the C, or carboxyl, terminus is on the right. The 20 most common, natural L-amino acids are alternatively designated by three-letter or one-letter codes. Peptides, as used herein, are considered to include "peptide analogs", structural modifications containing one or more modifications to L-amino acid side-chains or to the alpha-amino acid backbone. An example of a backbone modified peptide analog is the N-methyl glycine "peptoid" (Zuckermann et al., J. Amer. Chem. Soc. 114:10646-47 (1992)).

The term "cell penetrating peptide(s)" (CPP(s)) is defined as a carrier peptide that is capable of crossing biological membrane or a physiological barrier. Cell penetrating peptides are also called cell-permeable peptides, protein-transduction domains (PTD) or membrane-translocation sequences (MTS). CPPs have the ability to translocate *in vitro* and/or *in vivo* the mammalian cell membranes and enter into cells, and directs a conjugated compound of interest, such as a drug or marker, to a desired cellular destination, *e.g*. into he cytoplasm (cytosol, endoplasmic reticulum, Golgi apparatus, etc.) or the nucleus. Accordingly, the CPP can direct or facilitate penetration of a compound of interest across a phospholipid, mitochondrial, endosomal or nuclear membrane. The CPP can also direct a compound of interest from outside the cell through the plasma membrane, and into the cytoplasm or to a desired location within the cell, *e.g.*, the nucleus, the ribosome, the mitochondria, the endoplasmic reticulum, a lysosome, or a peroxisome. Alternatively or in addition, the CPP can direct a compound of interest across the blood-brain, trans-mucosal, hematoretinal, skin, gastrointestinal and/or pulmonary barriers.

Penetration across a biological membrane or a physiological barrier can be determined by various processes, for example by a cell penetration test having a first incubation step for the CPP conjugated to a marker in the presence of culture cells, followed by a fixating step, and then revelation of the presence of the marked peptide inside the cell. In another embodiment, the revelation step can be done with an incubation of the CPP in the presence of labeled antibodies and directed against the CPP, followed by detection in the cytoplasm or in immediate proximity of the cell nucleus, or even within it, of the immunologic reaction between the CPP's amino acid sequence and the labeled antibodies. Revelation can also be done by marking an amino acid sequence in the CPP and detecting the presence of the marking in the cell compartments. Cell penetration tests are well known to those skilled in the art. However, for example a cell penetration test was described in the above-mentioned patent application No WO 97/02840.

Several proteins and their peptide derivatives have been found to possess cell internalization properties including but not limited to the Human Immunodeficency Virus type 1 (HIV-1) protein Tat (Ruben et al. J. Virol. 63, 1-8 (1989)), the herpes virus tegument protein VP22 (Elliott and O'Hare, Cell 88, 223-233 (1997)), the homeotic protein of *Drosophila melanogaster* Antennapedia (the CPP is called Penetratin) (Derossi et al., J. Biol. Chem. 271, 18188-18193 (1996)), the protegrin 1 (PG-1) anti-microbial peptide SynB (Kokryakov et al., FEBS Lett. 327, 231-236 (1993)) and the basic fibroblast growth factor (Jans, Faseb J. 8, 841-847 (1994)). A number of other proteins and their peptide derivatives have been found to possess similar cell internalization properties. The carrier peptides that have been derived from these proteins show little sequence homology with each other, but are all highly cationic and arginine or lysine rich. Indeed, synthetic poly-arginine peptides have been shown to be internalized with a high level of efficiency (Futaki et al., J. Mol. Recognit. 16, 260-264 (2003); Suzuki et al., J. Biol. Chem. (2001)).

CPP can be of any length. For example CPP is less than or equal to 500, 250, 150, 100, 50, 25, 10 or 6 amino acids in length. For example CPP is greater than or equal to 4, 5, 6, 10, 25, 50, 100, 150 or 250 amino acids in length. The suitable length and design of the CPP will be easily determined by those skilled in the art. As general references on CPPs it can be cited: CELL PENETRATING PEPTIDES: PROCESSES AND APPLICATIONS, edited by Ulo Langel (2002); or Advanced Drug Delivery Reviews 57:489-660 (2005); or Dietz and Bähr, Moll Cell. Neurosci. 27:85-131 (2004).

In preferred embodiments the CPP is 4, 5, 6, 7, 5, 6, 7, 8, 9, 10, 11, 12, 13, 14, 15, 16, 17, 18, 19, 20, 21, 22, 23, 24 or 25 amino acids in length.

The cell penetrating peptides according to the invention can be, but not limited to, those described below or variants thereof. A "variant" that is at least about 50%, preferably at least about 70%, more preferably at least about 80%-85%, preferably at least about 90% and most preferably at least about 95%-99% identical thereto. For example, peptides can have substitutions at 1, 2, 3, 4 or more residues. The CPP can be used in their natural form (such as described above) or polymer form (dimer, trimer, etc.).

**Table 1: Selection of well-known cell penetrating peptide used for cargo delivery**

| SEQ ID NO: | Cell Penetrating Peptides | Amino acid sequences (Nter to Cter) in one letter code |
|---|---|---|
| 1 | Buforin II | TRSSRAGLQFPVGRVHRLLRK |
| 2 | DPV3 | RKKRRRESRKKRRRES |
| 3 | DPV6 | GRPRESGKKRKRKRLKP |
| 4 | DPV7 | GKRKKKGKLGKKRDP |
| 5 | DPV7b | GKRKKKGKLGKKRPRSR |
| 6 | DPV3/10 | RKKRRRESRRARRSPRHL |
| 7 | DPV10/6 | SRRARRSPRESGKKRKRKR |
| 8 | DPV1047 | VKRGLKLRHVRPRVTRMDV |
| 9 | DPV1048 | VKRGLKLRHVRPRVTRDV |
| 10 | DPV10 | SRRARRSPRHLGSG |
| 11 | DPV15 | LRRERQSRLRRERQSR |
| 12 | DPV15b | GAYDLRRRERQSRLRRRERQSR |
| 13 | GALA | WEAALAEALAEALAEHLAEALAEALEALAA |

| | Haptotactic peptides | |
|---|---|---|
| 14 | Cβ | KGSWYSMRKMSMKIRPFFPQQ |
| 15 | preCγ | KTRYYSMKKTTMKIIPFNRL |
| 16 | CαE | RGADYSLRAVRMKIRPLVTQ |
| 17 | hCT(9-32) | LGTYTQDFNKFHTFPQTAIGVGAP |
| 18 | HN-1 | TSPLNIHNGQKL |
| 19 | Influenza virus nucleoprotein (NLS) | NSAAFEDLRVLS |
| 20 | KALA | WEAKLAKALAKALAKHLAKALAKALKACEA |
| 21 | K-FGF | AAVALLPAVLLALLAP |
| 22 | Ku70 | VPMLKPMLKE |
| 23 | MAP | KLALKLALKALKAALKLA |
| 24 | MPG | GALFLGFLGAAGSTMGAWSQPKKKRKV |
| 25 | MPM (IP/K-FGF) | AAVALLPAVLLALLAP |
| 26 | N50 (NLS of NF-κB P50) | VQRKRQKLM |
| 27 | Pep-1 | KETWWETWWTEWSQPKKKRKV |
| 28 | Pep-7 | SDL WEMMMVSLACQY |
| 29 | Penetratin | RQIKIWFQNRRMKWKK |
| 30 | Short Penetratin | RRMKWKK |
| 31 | Poly Arginine - R₇ | RRRRRRR |
| 32 | Poly Arginine - R₉ | RRRRRRRRR |
| 33 | pISL | RVIRVWFQNKRCKDKK |
| 34 | Prion mouse PrPc₁₋₂₈ | MANLGYWLLALFVTMWTDVGLCKKRPKP |
| 35 | pVEC | LLIILRRRIRKQAHAHSK |
| 36 | SAP | VRLPPPVRLPPPVRLPPP |
| 37 | SV-40 (NLS) | PKKKRKV |
| 38 | SynB1 | RGGRLSYSRRRFSTSTGR |
| 39 | SynB3 | RRLSYSRRRF |
| 40 | SynB4 | AWSFRVSYRGISYRRSR |
| 41 | Tat₄₇₋₆₀ | YGRKKRRQRRRPPQ |
| 42 | Tat₄₇₋₅₇ | YGRKKRRQRRR |
| 43 | Tat₄₉₋₅₇ | RKKRRQRRR |
| 44 | Transportan | GWTLNSAGYLLGKINLKALAALAKKIL |
| 45 | Transportan 10 | AGYLLGKINLKALAALAKKIL |
| 46 | Transportan derivatives: | GWTLNSAGYLLG |
| 47 | | INLKALAALAKKIL |
| 48 | VP22 | DAATATRGRSAASRPTERPRAPARSASRPRRPVD |
| 49 | VT5 | DPKGDPKGVTVTVTVTVTGKGDPKPD |
| 50 | [Dmt¹]DALDA | Dmt-DRFK |

If necessary, several well known chemical strategies can be used by one skilled in the art for transforming a CPP into a drug candidate with increased stability *in vivo,* bioavailability and/or biological activity; such as:
- N- and C-terminus modifications to prevent exopeptidase degradation:
   o C-terminal amidation
   o N-terminal acetylation increases peptide lipophiliocity,
- cyclization by forming a disulfide bridge,
- alkylation of amide nitrogen to prevent endopeptidase degradation,
- introduction of non-natural amino acids to modify the recognition site of the endopeptidase (2-methylalanine, alpha-dialkylated glycine, oligocarbamate, oligourea, guanidino or amidino backbones...),
- incorporation of non-genetically encoded amino acids (methylation, halogenation or chlorination of glycine or phenylalanine) into the CPP amino acid sequence,
- replacement of some or even all the L-amino acids with their corresponding D-amino acid or beta-amino acid analogues. Such peptides may be synthesized as "inverso" or "retro-inverso" forms, that is, by replacing L-amino acids of the sequence with D-amino acids, or by reversing the sequence of the amino acids and replacing the L-amino acids with D-amino acids. Structurally, the retro-inverse peptide is much more similar to the original peptide than the simple D-analogue. D-peptides are substantially more resistant to peptidases, and therefore are more stable in serum and tissues compared to their L-peptide counterparts. In a preferred embodiment CPPs containing L-amino acids are capped with a single D-amino acid to inhibit exopeptidase destruction,
- synthesis of CPP-derived oligocarbamate; the oligocarbamate backbone consists of a chiral ethylene backbone linked through relatively rigid carbamate groups (Cho et al., Science 261:1303-1305 (1993)).

In another embodiment, the CPP contains contiguous or non- contiguous basic amino acid or amino acid analog, particularly guanidyl or amidinyl moieties. The terms "guanidyl" and "guanidine" are used interchangeably to refer to a moiety having the formula - HN=C(NH₂)NH (unprotonated form). As an example, arginine contains a guanidyl (guanidino) moiety, and is also referred to as 2-amino-5-guanidinovaleric acid or a-amino-6-guanidinovaleric acid. The terms "amidinyl" and "amidino" are used interchangeably and refer to a moiety having the formula -C(=NH)(NH2). A "basic amino acid or amino acid analog" has a side chain pKa of greater than 10. Preferred highly basic amino acids are histidine, arginine and/or lysine.

In a preferred embodiment, the CPP according to the invention comprises at least 1, 2, 3, 4, 5, 6, 7, 8, 9, 10 basic amino acid or amino acid analog, particularly lysine or arginine.

According to a more preferred embodiment, the CPP is further characterized by its ability to react with or bind to glycosaminoglycans (GAGs) (long unbranched molecules containing a repeating disaccharide unit) or specifically hyaluronic acid, heparin, heparan sulfate, dermatan sulfate, keratin sulfate or chondroitin sulfate and their derivatives. "Heparin, heparan sulfate or chondroitin sulfate derivatives" or "glycosaminoglycans" are understood to mean any product or sub-product as defined in the publications cited in references (Cardin and Weintraub, Arteriosclerosis 9: 21 (1989); Merton et al., Annu. Rev. Cell Biol. 8: 365 (1992); David, FASEB J. 7: 1023 (1993)).

The capacity of the CPPs to react with / bind to glycosaminoglycans (GAGs) can be determined by direct or indirect glycosaminoglycan-binding assays known in the art, such as the affinity co-electrophoresis (ACE) assay for peptide glycosaminoglycan binding described in the PCT patent application WO 00/45831. Several other methods well known in the art are available for analyzing GAG-peptides interactions, for example the method described in the PCT patent application WO 01/64738 or by Weisgraber and Rall (J. Biol. Chem., 262(33):11097-103) (specific example with the apolipoprotein B-100); or by a modified ELISA test: 96-well plates are coated with specific GAG (chondroitin sulfate A, B and C, heparin, heparan sulfate, hyaluronic acid, keratan sulfate, syndecan), peptide conjugated to a marker is then added for a defined time; after extensive washing, peptide binding is determined using specific analysis related to the marker.

CPPs capable of reacting *in vitro* and/or *in vivo* with glycosaminoglycans were described in the patent applications No WO O1/64738 and No WO 05/016960 and by De Coupade et al. (Biochem J. 390:407-18 (2005)). These peptides are amino acid sequences originating from human heparin binding proteins and/or anti-DNA antibodies selected from the group comprising: the lipoproteins such as human apolipoprotein B or E (Cardin et al., Biochem. Biosphys. Res. Com. 154: 741 (1988)), the agrine (Campanelli et al., Development 122: 1663-1672 (1996)), the insulin growth factor binding protein (Fowlkes et al., Endocrinol. 138: 2280-2285 (1997)), the human platelet-derived growth factor (Maher et al., Mol. Cell. Biol. 9: 2251-2253 (1989)), the human extracellular superoxide dismutase (EC-SOD) (Inoue et al., FEBS 269: 89-92 (1990)), the human heparin-binding epidermal growth factor-like growth factor (HB-EGF) (Arkonac et al., J. Biol. Chem. 273: 4400-4405 (1998)), the acid fibroblast growth factor (aFGF) (Fromm et al., Arch. Biochem. Bioph. 343: 92 (1997)), the basic fibroblast growth factor (bFGF) (Yayon et al., Cell 64: 841-848 (1991)), the human intestinal mucin 2 sequence (Xu et al., Glyconjug J. 13: 81-90 (1996)), the human gamma interferon (Lortat-Jacob & Grimaud, FEBS 280: 152-154 (1991)), the subunit p40 of human interleukin 12 (Hasan et al., J. Immunol. 162: 1064-1070 (1999)), the factor 1-alpha derived from stromal cells (Amara et al., J. Biol. Chem. 272: 200-204 (1999)), the human neutrophil derived "heparin binding protein" (CAP 37/azurocidin) (Pohl et al., FEBS 272: 200-204 (1990)), an immunoglobulin molecule such as CDR2 and/or CDR3 regions of the anti-DNA monoclonal murine antibody F4.1 (Avrameas et al., Proc. Natl. Acad. Sci. 95: 5601 (1998)), the hyper variable CDR3 region of human anti-DNA monoclonal antibody RTT79 (Stevenson et al., J. Autoimmunity 6: 809 (1993)), the hyper variable area CDR2 and/or CDR3 of the human anti-DNA monoclonal antibody NE-1 (Hirabayashi et al., Scand. J. Immunol. 37: 533 (1993)), the hypervariable area CDR3 of the human anti-DNA monoclonal antibody RT72 (Kalsi et al., Lupus 4: 375 (1995)).

According to a more preferred embodiment, the CPP comprises an amino-acid sequence selected from the group consisting of a) (XBBBXXBX)n ; b) (XBBXBX)n ;c) (BBXmBBXp)n ; d) (XBBXXBX)n; e) (BXBB)n and f) (an antibody fragment), wherein each B is independently a basic amino acid preferably lysine or arginine; each X is independently a non-basic amino acid preferably hydrophobic amino acid, such as alanine, isoleucine, leucine, methionine, phenylalanine, tryptophan, valine or tyrosine; each m is independently an integer from zero to five; each n is independently an integer between one and ten; and each p is independently an integer between zero to five. In certain embodiments n may be 2 or 3 and X may be a hydrophobic amino acid. An antibody fragment is meant to include a less than full-length immunoglobulin polypeptide, *e.g*., a heavy chain, light chain, Fab, Fab'2, Fv or Fc. The antibody can be for example human or murine. Preferably the antibody is an anti-DNA antibody. Preferably, the antibody fragment contains all or part of the CDR2 region of an antibody, particularly at least a portion of at least 6 or 10 amino acids in length of a CDR2 region of an anti-DNA antibody. Alternatively, the antibody contains all or part of the CDR3 region of an antibody, particularly at least a portion of at least 6 or 12 amino acids in length of a CDR3 region of an anti-DNA antibody. More specifically, the antibody fragment contains at least one CDR3 region of an anti-DNA human antibody, such as RTT79, NE-1 and RT72. Such antibody fragments have been described in PCT patent application n° WO 99/07414. More preferably the antibody has specific ligand-recognition (*i.e.* targeting) properties to achieve cell-type-specific nucleic acid delivery.

Preferably, the CPP according to the invention is further characterized in that it is originating from human proteins (*i.e.,* proteins naturally expressed by human cells). Thus, the characteristic of CPPs derived from human proteins compared to the CPPs derived from non-human proteins, is of primary interest in the planned use of these CPPs, since their immunogenicity is avoided or lowered. In addition, De Coupade et al., (Biochem J. 390:407-18 (2005)) have shown that human-derived peptides have low *in vivo* toxicity profiles consistent with their use as therapeutic delivery systems, unlike existing carrier peptides such as Tat peptides (Trehin and Merkle, Eur. J. Pharm. Biopharm. 58, 209-223 (2004)).

Among the CPPs described above, preferred are those capable of specifically penetrating into the cytoplasm. Penetration of a CPP into the cytoplasm can be determined by various processes *in vitro* well known by one skilled in the art: for example by incubating the CPP with cells; then, the cells are incubated in the presence of specific anti-CPP labeled antibodies and specific anti-cytoplasm protein labeled antibodies, followed by detection in the cytoplasm of the immunologic reaction between the CPP and the labeled antibodies. Another method is to conjugate the CPP to colloidal gold and incubate the conjugate with cells. The cells are then treated as usual for the electron microscope to visualise the intracellular localization.

Accordingly, preferred CPPs, derived from human heparin binding protein and capable of specifically penetrate into the cytoplasm of a target cell are selected from the group comprising:
- DPV3 (SEQ ID NO: 2): CPP reacting with heparin and dimer of a peptide derived from the C-terminal part of the sequence of human extracellular superoxide dismutase (EC-SOD) (Inoue et al., FEBS 269: 89-92 (1990)). It comprises an amino acid sequence of formula c) (BBXmBBXp)n wherein m = 0, p = 0 and n = 1;
- DPV6 (SEQ ID NO: 3): CPP reacting with heparin and derived from the amino acid sequence of the C-terminal part of chain A of the human platelet-derived growth factor (Maher et al., Mol. Cell. Biol. 9: 2251-2253 (1989)). It comprises an amino acid sequence of formula c) (BBXmBBXp)n wherein m = 0, p = 0 and n = 1;
- DPV7 (SEQ ID NO: 4) and DPV7b (SEQ ID NO: 5): CPPs reacting with heparin and derived from the C-terminal part of the sequence of the human heparin-binding epidermal growth factor-like growth factor (HB-EGF) (Arkonac et al., J. Biol. Chem. 273: 4400-4405 (1998)). Both CPP comprise an amino acid sequence of formula c) (BBXmBBXp)n wherein m=0,p=0 and n= 1;
- DPV 10 (SEQ ID NO: 10): CPP reacting with heparin and corresponding to the C-terminal part of the human intestinal mucin 2 sequence (Xu et al., Glyconjug J. 13: 81-90 (1996)). It comprises an amino acid sequence of formula e) (BXBB)n wherein n = 1;
- DPV3/10 (SEQ ID NO: 6): CPP reacting with heparin and derived from the C-terminal part of the sequence of human extracellular superoxide dismutase (EC-SOD) (see above) and from C-terminal part of the human intestinal mucin 2 sequence (see above). It comprises an amino acid sequence of formula c) (BBXmBBXp)n wherein m = 1, p = 2 and n = 1;
- DPV10/6 (SEQ ID NO: 7): CPP reacting with heparin and derived from the C-terminal part of the human intestinal mucin 2 sequence (see above) and from the C-terminal part of chain A of the platelet-derived growth factor (see above). It comprises an amino acid sequence of formula c) (BBXmBBXp)n wherein m = 1, p = 2 and n = 1;
- DPV 1047 (SEQ ID NO: 8): CPP reacting with heparin derived from the amino acid sequence (3358-3372) of the human lipoprotein B (Cardin et al., Biochem. Biosphys. Res. Com. 154: 741 (1988)) and from the sequence of the peptide corresponding to the hypervariable area CDR3 of the human anti-DNA monoclonal antibody NE-1 (Hirabayashi et al., Scand. J. Immunol. 37: 533 (1993)). It comprises an amino acid sequence of formula d) (XBBXXBX)n wherein n = 1, and an antibody fragment or an amino acid sequence of formula b) (XBBXBX)n wherein n = 1;
- DPV15b (SEQ ID NO: 11): CPP reacting with heparin and containing part of the sequence of the "heparin binding protein" (CAP 37). It comprises an amino acid sequence of formula d) (XBBXXBX) repeated twice.

In a preferred embodiment, one cysteine residue is added to the amino acid sequence of the CPPs, preferably to either the C- or N-terminus. The cysteine residue provides a free sulfhydryl group to allow conjugation of the CPPs to the nucleic acids.

The term "hydrophilic polymer" means any polymer that has an affinity for water. It generally includes polar groups. Hydrophilic polymers are well known from one skilled in the art; they comprise polyalkyl glycols, polysaccharides, polyols, polycarboxylates, or poly(hydro)ester, and specifically polyethylene glycol or poly[N-(2-hydroxypropyl)methacrylamide] (pHPMA).

As used herein, the term "PEG" represents certain polyethylene glycol containing substituents having the designated number of ethylene glycol subunits. The term "PEG(z)" represents polyethylene glycol containing "z" ethylene glycol subunits. The term PEG also includes PEG-polymer of polyethylene glycol units; the polymer being linear, multiarmed or branched.

The term "polyethylene glycol-based linker" or "PEG-based linker" refers to a crosslinker having a structure according to formula II: - 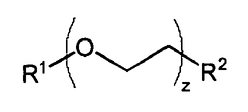 in which
z is an integer from 1 to 10.000, preferably from 1 to 500, by way of example from 1 to 100 or 1 to 50 or 1 to 20 and more preferably from 1 to 10..
R¹ and R² are divalent organic radicals independently selected from substituted or unsubstituted alkyl, substituted or unsubstituted heteroalkyl and substituted or unsubstituted aryl.

Preferably R¹ and R² are independently selected from =O, SR³, -NHR³ and -OR³, in which R³ is H, substituted or unsubstituted alkyl, substituted or unsubstituted heteroalkyl, acyl, -OR⁴, -C(O)R⁴, -C(O)OR⁴, -C(O)NR⁴R⁵, -P(O)(OR⁴)₂, -C(O)CHR⁴R⁵, -NR⁴R⁵, -N(+)R⁴R⁵R⁶, -SR⁴ or SiR⁴R⁵R⁶. The symbols R⁴, R⁵ and R⁶ independently represent H, substituted or unsubstituted alkyl, substituted or unsubstituted heteroalkyl and substituted or unsubstituted aryl, wherein R⁴ and R⁵ together with the nitrogen atom to which they are attached are optionally joined to form a substituted or unsubstituted heterocycloalkyl ring system having from 4 to 6 members, optionally containing two or more heteroatoms.

Advantageously, the PEG-based linker according to the invention is flexible, non immunogenic, not susceptible to cleavage by proteolytic enzymes and enhances the solubility in aqueous media of the CPP-nucleic acid conjugates.

In another embodiment, the PEG enhances the solubility in aqueous media of the PEG nucleic acid conjugates.

The term "alkyl" by itself or as part of another substituent, means, unless otherwise stated, a straight or branched chain, or cyclic hydrocarbon radical, or combination thereof, which may be fully saturated, mono-or polyunsaturated and can include di- and multivalent radicals, having the number of carbon atoms designated (i.e. C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl) methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2- (butadienyl), 2,4-pentadienyl, 3 (1,4-pentadienyl), ethynyl,1-and 3-propynyl, 3-butynyl, and the higher homologs and isomers. The term "alkyl" unless otherwise noted, is also meant to include those derivatives of alkyl defined in more detail below, such as "heteroalkyl". Alkyl groups, which are limited to hydrocarbon groups are termed "homoalkyl".

The term "alkylene" by itself or as part of another substituent means a divalent radical derived from an alkane, as exemplified, but not limited, by -CH₂-CH₂-CH₂-CH₂-, and further includes those groups described below as "heteroalkylene". Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "heteroalkyl" by itself or in combination with another term, means, unless otherwise stated, a stable straight or branched chain, or cyclic hydrocarbon radical, or combinations thereof, consisting of the stated number of carbon atoms and at least one heteroatom selected from the group consisting of O, N, Si and S, and wherein the nitrogen, carbon and sulfur atoms may optionally be oxidized and the nitrogen heteroatom may optionally be quaternized. The heteroatom (s) O, N and S and Si may be placed at any interior position of the heteroalkyl group or at the position at which the alkyl group is attached to the remainder of the molecule. Examples include, but are not limited to,-CH₂, CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si (CH₃)₃, -CH₂-CH=N-OCH₃ and CH=CH-N(CH₃)-CH₃. Up to two heteroatoms may be consecutive, such as, for example, -CH₂-NH-OCH₃ and -CH₂-O-S-(CH₃)₃. Similarly, the term "heteroalkylene" by itself or as part of another substituent means a divalent radical derived from heteroalkyl, as exemplified, but not limitedby,-CH₂-CH₂-S-CH₂-CH₂-and-CH₂-S-CH₂-CH₂-NH-CH₂-. For heteroalkylene groups, heteroatoms can also occupy either or both of the chain termini (*e.g.,* alkyleneoxy, alkylenedioxy, alkyleneamino, alkylenediamino, and the like). The terms "heteroalkyl" and "heteroalkylene" encompass poly(ethylene glycol) and its derivatives (see, for example, Shearwater Polymers Catalog, 2001). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-.

The term "lower" in combination with the terms "alkyl" or "heteroalkyl" refers to a moiety having from 1 to 6 carbon atoms.

The terms "alkoxy", "alkylamino" and "alkylthio" (or thioalkoxy) are used in their conventional sense, and refer to those alkyl groups attached to the remainder of the molecule via an oxygen atom, an amino group, or a sulfur atom, respectively.

In general, an "acyl substituent" is also selected from the group set forth above. As used herein, the term "acyl substituent" refers to groups attached to, and fulfilling the valence of a carbonyl carbon that is either directly or indirectly attached to the polycyclic nucleus of the compounds of the present invention.

The terms "cycloalkyl" and "heterocycloalkyl", by themselves or in combination with other terms, represent, unless otherwise stated, cyclic versions of substituted or unsubstituted "alkyl" and substituted or unsubstituted "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3morpholinyl, tetrahydrofuran-2-yl, tetrahydrofuran-3-yl,tetrahydrothien-2-yl, tetrahydrothien-3-yl, 1-piperazinyl, 2-piperazinyl, and the like. The heteroatoms and carbon atoms of the cyclic structures are optionally oxidized.

The terms "halo" or "halogen"by themselves or as part of another substituent, mean, unless otherwise stated, a fluorine, chlorine, bromine, or iodine atom. Additionally, terms such as "haloalkyl" are meant to include monohaloalkyl and polyhaloalkyl. For example, the term "halo(C₁-C₄) alkyl" is mean to include, but not be limited to, trifluoromethyl, 2,2,2-trifluoroethyl, 4-chlorobutyl, 3-bromopropyl, and the like.

The term "aryl" means, unless otherwise stated, a substituted or unsubstituted polyunsaturated, aromatic, hydrocarbon substituent which can be a single ring or multiple rings (preferably from 1 to 3 rings) which are fused together or linked covalently. The term "heteroaryl" refers to aryl groups (or rings) that contain from one to four heteroatoms selected from N,O, and S, wherein the nitrogen, carbon and sulfur atoms are optionally oxidized, and the nitrogen atom (s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2pyrrolyl, 3-pyrrolyl, 3-pyrazolyl, 2-imidazolyl, 4-imidazolyl, pyrazinyl, 2-oxazolyl, 4oxazolyl, 2-phenyl-4-oxazolyl, 5-oxazolyl, 3-isoxazolyl, 4-isoxazolyl, 5-isoxazolyl, 2thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl,1-isoquinolyl, 5-isoquinolyl, 2-quinoxalinyl, 5-quinoxalinyl, 3-quinolyl, and 6-quinolyl.

Substituents for each of the above noted aryl and heteroaryl ring systems are selected from the group of acceptable substituents described below. "Aryl" and "heteroaryl" also encompass ring systems in which one or more non-aromatic ring systems are fused, or otherwise bound, to an aryl or heteroaryl system.

For brevity, the term "aryl" when used in combination with other terms (e.g., aryloxy, arylthioxy, arylalkyl) includes both aryl and heteroaryl rings as defined above.

Thus, the term "arylalkyl" is meant to include those radicals in which an aryl group is attached to an alkyl group (*e.g*., benzyl, phenethyl, pyridylmethyl and the like) including those alkyl groups in which a carbon atom (*e.g*., a methylene group) has been replaced by, for example, an oxygen atom (*e.g*., phenoxymethyl, 2-pyridyloxymethyl, 3-(1-naphthyloxy)propyl, and the like).

Each of the above terms (*e.g*., "alkyl", "heteroalkyl", "aryl" and "heteroaryl") include both substituted and unsubstituted forms of the indicated radical. Preferred substituents for each type of radical are provided below.

Substituents for the alkyl, and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generally referred to as "alkyl substituents" and "heteroalkyl substituents", respectively, and they can be one or more of a variety of groups selected from, but not limited to:-OR', =O, =NR', =N-OR',-NR'R", -SR', -halogen, -SiR'R"R"', -OC(O)R', -C (O)R', -CO₂R', -CONR'R", -OC(O)NR'R", -NR"C(O)R', -NR'-C(O)NR"R"', -NR"C(O)₂R', -NR-C(NR'R")=NR"", -NR-C(NR'R")=NR"', -S(O)R', -S(O)₂R', -S(O)₂NR'R", -NRSO₂R' NRR'SO₂R", -CN and -NO₂ in a number ranging from zero to (2m'+1), where m' is the total number of carbon atoms in such radical. R',R", R"' and R"" each preferably independently refer to hydrogen, substituted or unsubstituted heteroalkyl, substituted or unsubstituted aryl, *e.g*., aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R"' and R"" groups when more than one of these groups is present. When R' and R" are attached to the same nitrogen atom, they can be combined with the nitrogen atom to form a 5-, 6-, or 7-membered ring. For example, NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (*e.g*.,-CF₃ and-CH₂CF₃) and acyl (*e.g.*, -C (O)CH₃, -C(O)CF₃, C(O)CH₂OCH₃, and the like).

Similar to the substituents described for the alkyl radical, the aryl substituents and heteroaryl substituents are generally referred to as "aryl substituents" and "heteroaryl substituents", respectively and are varied and selected from, for example: halogen, -OR', =O, =NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R")=NR"', -S(O)R', -S(O)₂R', -S(O)₂NR'R", -NRSO₂R', -CN and -NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R"' and R"" are preferably independently selected from hydrogen,(C₁-C₈) alkyl and heteroalkyl, unsubstituted aryl and heteroaryl, (unsubstitutedaryl)-(C₁-C₄)alkyl, and (unsubstituted aryl)oxy-(C₁-C₄)alkyl. When a compound of the invention includes more than one R group, for example, each of the R groups is independently selected as are each R', R", R''' and R"" groups when more than one of these groups is present.

Two of the aryl substituents on adjacent atoms of the aryl or heteroaryl ring may optionally be replaced with a substituent of the formula -T-C(O)- (CRR')q-U-, wherein T and U are independently NR-,-O-,-CRR'-or a single bond, and q is an integer of from 0 to 3. Alternatively, two of the substituents on adjacent atoms of the aryl or heteroaryl ring may optionally be replaced with a substituent of the formula -A- (CH2)r-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula-(CRR')s-X-(CR"R"')d-, where s and d are independently integers of from 0 to 3, and X is -O-, -NR'-, -S-, -S(O)-, -S(O)₂-, or-S(O)₂NR'-. The substituents R, R', R" and R"' are preferably independently selected from hydrogen or substituted or unsubstituted (C₁-C₆) alkyl.

As used herein, the term "heteroatom" includes oxygen (O), nitrogen (N), sulfur (S) and silicon (Si).

The symbol "R" is a general abbreviation that represents a substituent group that is selected from substituted or unsubstituted alkyl, substituted or unsubstituted heteroalkyl, substituted or unsubstituted aryl, substituted or unsubstituted heteroaryl, and substituted or unsubstituted heterocyclyl groups.

In a preferred embodiment, the PEG-based linker has the formula III: 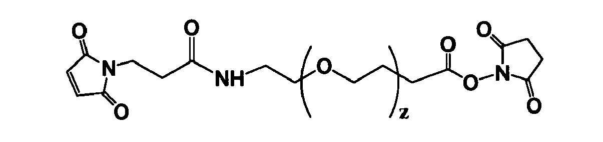 wherein z (representing the number of ethylene glycol subunits) is an integer from 1 to 10.000, preferably from 1 to 500, more preferably from 1 to 100.
By way of example, if z = 4, then the PEG-based linker has the formula IV:

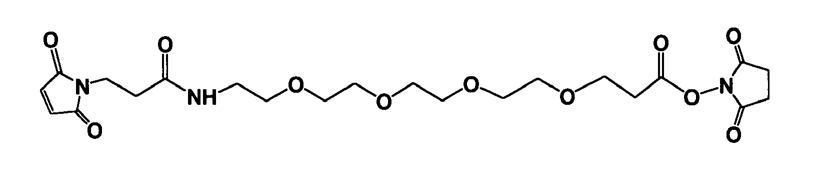

The formula V below shows the PEG-based linker of formula III once linked to an amino acid sequence (*e.g*. a CPP) and a nucleic acid molecule (*e.g*. a siRNA): 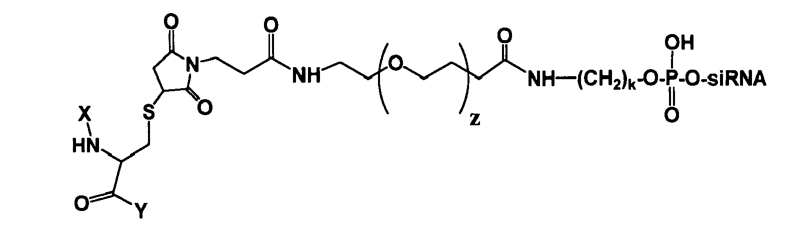 wherein:
z is an integer from 1 to 10.000, preferably from 1 to 500, more preferably from 1 to 100;
k is an integer from 1 to 250, preferably from 1 to 100 and more preferably from 1 to 10;
X = H, CO(CH₃), any amino acid or an amino acid sequence
Y = H, OH, NH₂, any amino acid or an amino acid sequence

The term "nucleoside" refers to a molecule having a purine or pyrimidine base covalently linked to a ribose or deoxyribose sugar. Exemplary nucleosides include adenosine, guanosine, cytidine, uridine and thymidine. The term "nucleotide" refers to a nucleoside having one or more phosphate groups joined in ester linkages to the sugar moiety. Exemplary nucleotides include nucleoside monophosphates, diphosphates and triphosphates. The term "nucleotide analog", also referred to herein as an "altered nucleotide" or "modified nucleotide" refers to a non-standard nucleotide, including non-naturally occurring ribonucleotides or deoxyribonucleotides. Preferred nucleotide analogs are modified at any position so as to alter certain chemical properties of the nucleotide yet retain the ability of the nucleotide analog to perform its intended function. The terms "nucleotide" and "nucleotide analog" can be used interchangeably.

The term "oligonucleotide" ("ON") refers to a short polymer of nucleotides and/or nucleotide analogs.

The term "nucleic acid analog(s)" refers to structurally modified, polymeric analogs of DNA and RNA made by chemical synthesis from monomeric nucleotide analog units, and possessing some of the qualities and properties associated with nucleic acid.

The term "RNA" or "RNA molecule" or "ribonucleic acid molecule" refers to a polymer of ribonucleotides. The term "DNA" or "DNA molecule" or "deoxyribonucleic acid molecule" refers to a polymer of deoxyribonucleotides. DNA and RNA can be synthesized naturally *(e.g.,* by DNA replication or transcription of DNA, respectively). RNA can be posttranscriptionally modified. DNA and RNA can also be chemically synthesized. DNA and RNA can be single-stranded (*i.e*., ssRNA and ssDNA, respectively) or multi-stranded (*e.g*., double stranded, *i.e.*, dsRNA and dsDNA, respectively). "mRNA" or "messenger RNA" is single-stranded RNA that encodes the amino acid sequence of one or more polypeptide chains. This information is translated during protein synthesis when ribosomes bind to the mRNA.

The terms "polynucleotide(s)", "nucleic acid(s)" or "nucleic acid molecule(s)" are used interchangeably herein and refer to a polymer of nucleotides joined together by a phosphodiester linkage between 5' and 3' carbon atoms. Polynucleotide(s), nucleic acid(s) or nucleic acid molecule(s) and their analogs can be linear, circular, or have higher orders of topology (*e.g.*, supercoiled plasmid DNA). DNA can be in the form of antisense, plasmid DNA, parts of a plasmid DNA, vectors (*e.g.*, P1-derived Artificial Chromosome, Bacterial Artificial Chromosome, Yeast Artificial Chromosome, or any artificial chromosome), expression cassettes, chimeric sequences, chromosomal DNA, or derivatives of these groups. RNA can be in the form of oligonucleotide RNA, tRNA (transfer RNA), snRNA (small nuclear RNA), rRNA (ribosomal RNA), mRNA (messenger RNA), antisense RNA, (interfering) double-stranded and single-stranded RNA, ribozymes, chimeric sequences, or derivatives of these groups. Nucleic acids can be single ("ssDNA"), double ("dsDNA"), triple ("DNA"), or quadruple ("qsDNA") stranded DNA, and single stranded RNA ("RNA") or double stranded RNA ("dsRNA"). "Multistranded" nucleic acid contains two or more strands and can be either homogeneous as in double stranded DNA, or heterogeneous, as in DNA/RNA hybrids. Multistranded nucleic acid can be full length multistranded, or partially multistranded. It can further contain several regions with different numbers of nucleic acid strands. Partially single stranded DNA is considered a sub-group of ssDNA and contains one or more single stranded regions as well as one or more multiple stranded regions.

The term "plasmid DNA" refers to a circular double-stranded DNA construct used as a cloning vector, and which forms an extrachromosomal genetic element in some eukaryotes or integrates into the host chromosomes.

As used herein, the term "small interfering RNA" ("siRNA") (also referred to in the art as "short interfering RNAs") refers to a RNA (or RNA analog) comprising between about 10-50 nucleotides (or nucleotide analogs) which is capable of directing or mediating RNA interference.

The term "RNA analog" refers to an polynucleotide (*e.g*., a chemically synthesized polynucleotide) having at least one altered or modified nucleotide as compared to a corresponding unaltered or unmodified RNA but retaining the same or similar nature or function as the corresponding unaltered or unmodified RNA. As discussed above, the oligonucleotides may be linked with linkages which result in a lower rate of hydrolysis of the RNA analog as compared to an RNA molecule with phosphodiester linkages. For example, the nucleotides of the analog may comprise methylenediol, ethylene diol, oxymethylthio, oxyethylthio, oxycarbonyloxy, phosphorodiamidate, phophoroamidate, and/or phosphorothioate linkages. Exemplary RNA analogues include sugar-and/or backbone-modified ribonucleotides and/or deoxyribonucleotides. Such alterations or modifications can further include addition of non-nucleotide material, such as to the end (s) of the RNA or internally (at one or more nucleotides of the RNA). RNA analog needs only to be sufficiently similar to natural RNA that it has the ability to mediate (mediates) RNA interference.

As used herein, the term "RNA interference" ("RNAi") refers to a selective intracellular degradation of RNA. RNAi occurs in cells naturally to remove foreign RNAs (*e.g*., viral RNAs). Natural RNAi proceeds via fragments cleaved from free dsRNA which direct the degradative mechanism to other similar RNA sequences.

A siRNA having a "sequence sufficiently complementary to a target mRNA sequence to direct target-specific RNA interference (RNAi)" means that the siRNA has a sequence sufficient to trigger the destruction of the target mRNA by the RNAi machinery or process, i.e. there is preferably greater than 80% sequence identity, or more preferably greater than 90% 91%, 92%, 93%, 94%, 95%, 96%, 97%, 98%, 99%, or even 100% sequence identity, between the siRNA and the portion of the target mRNA sequence encoded by the target gene.

The term "cleavage site" refers to the residues, *e.g*., nucleotides, at which RISC* cleaves the target RNA, *e.g*., near the center of the complementary portion of the target RNA, e. g., about 8-12 nucleotides from the 5' end of the complementary portion of the target RNA.

The term "upstream of the cleavage site" refers to residues, *e.g*., nucleotides or nucleotide analogs, 5' to the cleavage site. Upstream of the cleavage site with reference to the antisense strand refers to residues, *e.g*, nucleotides or nucleotide analogs 5' to the cleavage site in the antisense strand.

The term "downstream of the cleavage site" refers to residues, *e.g*., nucleotides or nucleotide analogs, located 3' to the cleavage site. Downstream of the cleavage site with reference to the antisense strand refers to residues, *e.g*., nucleotides or nucleotide analogs, 3' to the cleavage site in the antisense strand.

The term "mismatch" refers to a basepair consisting of noncomplementary bases, *e.g*. not normal complementary G:C, A:T or A:U base pairs.

The term "phosphorylated" means that at least one phosphate group is attached to a chemical (*e.g*., organic) compound. Phosphate groups can be attached, for example, to proteins or to sugar moieties via the following reaction: free hydroxyl group + phosphate donor gives phosphate ester linkage. The term "5' phosphorylated" is used to describe, for example, polynucleotides or oligonucleotides having a phosphate group attached via ester linkage to the C5 hydroxyl of the 5' sugar (*e.g*., the 5' ribose or deoxyribose, or an analog of same). Mono-, di-, and tri-phosphates are common. Also intended to be included within the scope of the invention are phosphate group analogs which function in the same or similar manner as the mono-, di-, or triphosphate groups found in nature (see *e.g*., exemplified analogs).

A target gene is a gene targeted by a compound of the invention (*e.g*., a siRNA (targeted siRNA), candidate siRNA derivative, siRNA derivative, modified siRNA, etc.), *e.g*., for RNAi-mediated gene knockdown. One portion of a siRNA is complementary (*e.g*., fully complementary) to a section of the mRNA of the target gene.

Various aspects of the nucleic acid in the scope of the invention are described in further detail in the following subsections.

### I. Antisense-oligonucleotides (AS-ONs)

According to a first aspect, the present invention relates to cell penetrating peptide-antisense oligonucleotide conjugates (CPP-AS-ONs).

AS-ONs usually consist of 15-20 nucleotides, which are complementary to their target mRNA. Two major mechanisms contribute to their antisense activity. The first is that most AS-ONs are designed to activate RNase H, which cleaves the RNA moiety of a DNA/RNA heteroduplex and therefore leads to degradation of the target mRNA. In addition, AS-ONs that do not induce RNase H cleavage can be used to inhibit translation by steric blockade of the ribosome. When the AS-ONs are targeted to the 5'-terminus, binding and assembly of the translation machinery can be prevented. Furthermore, AS-ONs can be used to correct aberrant splicing.

In general, three types of modifications of ribonucleotides can be distinguished:
analogs with unnatural bases, modified sugars (especially at the 2' position of the ribose) or altered phosphate backbones.

A variety of heterocyclic modifications have been described, which can be introduced into AS-ONs to strengthen base-pairing and thus stabilize the duplex between AS-ONs and their target mRNAs. A comprehensive review dealing with base-modified ONs was published by Herdewijn in Antisense Nucleic Acids Drug Dev. 10:297-310 (2000).

Thus the invention relates to AS-ONs with modified sugar moieties and phosphate backbones as described below.

Phosphorothioate oligodeoxynucleotides are the major representatives of 'first generation' DNA analogs that are the best known and most widely used AS-ONs to date (reviewed in Eckstein, Antisense Nucleic Acids Drug Dev. 10:117-121 (2000)).

'Second generation' antisense-oligonucleotides contain nucleotides with alkyl modifications at the 2' position of the ribose. 2'-*O*-methyl and 2'-*O*-methoxy-ethyl RNA are the most important members of this class. AS-ONs made of these building blocks are less toxic than phosphorothioate DNAs and have a slightly enhanced affinity towards their complementary RNAs (Kurreck et al.,, Nucleic Acids Res. 30:1911-1918 (2002); Crooke et al., Biochem. J. 312:599-608 (1995)).

For most antisense approaches, target RNA cleavage by RNase H is desired in order to increase antisense potency. Therefore, 'gapmer technology' has been developed. Gapmers consist of a central stretch of DNA or phosphorothioate DNA monomers and modified nucleotides such as 2'-*O*-methyl RNA at each end. The modified ends prevent nucleolytic degradation of the AS-ON and the contiguous stretch of at least four or five deoxy residues between flanking 2'-*O*-methyl nucleotides was reported to be sufficient for activation of *Escherichia coli* and human RNase H, respectively (Crooke et al., Biochem. J. 312:599-608 (1995); Monia et al., J. Biol. Chem. 268:14514-14522 (1993); Wu et al., J. Biol. Chem. 274:28270-28278 (1999)).

'Third generation' of modified nucleotides have been developed to improve properties such as target affinity, nuclease resistance and pharmacokinetics. The concept of conformational restriction has been used widely to enhance binding affinity and biostability. DNA and RNA analogs with modified phosphate linkages or riboses as well as nucleotides with a completely different chemical moiety substituting the furanose ring have been developed. Examples of modified nucleotides with improved properties are described below, although further modifications (known from one skilled in the art) may prove to have a great potential as antisense molecules.
Peptide nucleic acids (PNAs). In PNAs the deoxyribose phosphate backbone is replaced by polyamide linkages. PNA can be obtained commercially, e.g. from Applied Biosystems (Foster City, CA, USA). PNAs have favorable hybridization properties and high biological stability, but do not elicit target RNA cleavage by RNase H. See for review Nielsen, Methods Enzymol. 313:156-164 (1999); Braasch and Corey, Biochemistry 41:4503-4509 (2002)
N3'-P5' phosphoroamidates (NPs). N3'-PS' phosphoroamidates (NPs) are another example of a modified phosphate backbone, in which the 3'-hydroxyl group of the 2'-deoxyribose ring is replaced by a 3'-amino group. NPs exhibit both a high affinity towards a complementary RNA strand and nuclease resistance (Gryaznov and Chen, J. Am. Chem. Soc. 116:3143-3144 (1994)). Their potency as antisense molecules has been demonstrated *in vivo,* where a phosphoroamidate ON was used to specifically down-regulate the expression of the *c-myc* gene (Skorski et al., Proc. Natl Acad. Sci. USA 94:3966-3971 (1997). The sequence specificity of phosphoroamidate-mediated antisense effects by steric blocking of translation initiation could be demonstrated in cell culture, and *in vivo* with a system in which the target sequence was present just upstream of the firefly luciferase initiation codon (Faira et al., Nat. Biotechnol. 19:40-44 (2001).
2'-Deoxy-2'-fluoro-beta-d-arabino nucleic acid (FANA). Oligonucleotides made of arabino nucleic acid, the 2' epimer of RNA, or the corresponding 2'-deoxy-2'-fluoro-beta-d-arabino nucleic acid analogue (FANA) were the first uniformly sugar-modified AS-ONs reported to induce RNase H cleavage of a bound RNA molecule (Damha et al., J. Am. Chem. Soc. 120:12976-12977 (1998)). The fluoro substituent is thought to project into the major groove of the helix, where it should not interfere with RNase H.
Locked nucleic acid (LNA). LNA is a ribonucleotide containing a methylene bridge that connects the 2'-oxygen of the ribose with the 4'-carbon (reviewed in Elayadi and Corey, Curr. Opinion Invest. Drugs 2:558-561 (2001); Braasch and Corey, Chem. Biol. 8:1-7 (2001); ∅rum and Wengel, Curr. Opinion Mol. Ther. 3:239-243 (2001)) Oligonucleotides containing LNA are commercially available from Proligo (Paris, France and Boulder, CO, USA). Introduction of LNA into a DNA oligonucleotide induces a conformational change of the DNA/RNA duplex towards the A-type helix (Bondensgaard et al., Chem. Eur. J. 6:2687-2695 (2000)) and therefore prevents RNase H cleavage of the target RNA. If degradation of the mRNA is intended, a chimeric DNA/LNA gapmer that contains a stretch of 7-8 DNA monomers in the center to induce RNase H activity should be used (Kurreck et al., Nucleic Acids Res. 30:1911-1918 (2002)). Chimeric 2'-O-methyl-LNA oligonucleotides that do not activate RNase H could, however, be used as steric blocks to inhibit intracellular gene-dependent *trans* activation and hence suppress gene expression (Arzumanov et al., Biochemistry 40:14645-14654 (2001)). Chimeric DNA/LNA oligonucleotides reveal an enhanced stability against nucleolytic degradation and an extraordinarily high target affinity (Kurreck et al., Nucleic Acids Res. 30:1911-1918 (2002); Wahlestedt et al., Proc. Natl Acad. Sci. USA 97:5633-5638 (2000)). Due to their high affinity for their complementary sequence, LNA oligonucleotides as short as eight nucleotides long are efficient inhibitors in cell extracts. Full LNA and chimeric DNA/LNA oligonucleotides offer an attractive set of properties, such as stability against nucleolytic degradation, high target affinity, potent biological activity and apparent lack of acute toxicity.
Morpholino oligonucleotides (MF). Morpholino ONs are nonionic DNA analogs, in which the ribose is replaced by a morpholino moiety and phosphoroamidate intersubunit linkages are used instead of phosphodiester bonds. They are commercially available from Gene Tools LLC (Corvallis, OR, USA). A review of their usage has been carried out by Heasman, Dev. Biol. 243:209-214 (2002); and In GENESIS, volume 30, issue 3, (2001). MFs do not activate RNase H and, if inhibition of gene expression is desired, they should therefore be targeted to the 5' untranslated region or to the first 25 bases downstream of the start codon to block translation by preventing ribosomes from binding.
Cyclohexene nucleic acids (CeNA). Replacement of the five-membered furanose ring by a six-membered ring is the basis for cyclohexene nucleic acids (CeNAs), which are characterized by a high degree of conformational rigidity of the oligomers. They form stable duplexes with complementary DNA or RNA and protect oligonucleotides against nucleolytic degradation (Wang et al., J. Am. Chem. Soc. 122:8595-8602 (2000)).
Tricyclo-DNA (tcDNA). Tricyclo-DNA (tcDNA) is another nucleotide with enhanced binding to complementary sequences (Steffens and Leumann, J. Am. Chem. Soc. 119:11548-11549 (1997); Renneberg and Leumann, J. Am. Chem. Soc. 124:5993-6002 (2002)). tcDNA does not activate RNase H cleavage of the target mRNA.

### II. Ribozymes and DNA enzymes

In another aspect, the present invention relates to cell penetrating peptide-ribozymes conjugates and to cell penetrating peptide-DNA enzyme conjugates.

Ribozymes are RNA enzyme that catalyses the reaction of a free substrate, *i.e*. possesses catalytic activity *in trans.* A variety of ribozymes, catalyzing intramolecular splicing or cleavage reactions, have been found in lower eukaryotes, viruses and some bacteria. The different types of ribozymes and their mechanisms of action have been described comprehensively (Eckstein and Lilley, CATALYTIC RNA, Springer Verlag, Berlin/Heidelberg/New York (1996); James and Gibson, Blood 91:371-382 (1998); Sun et al., Pharmacol. Rev. 52:325-347 (2000); Jen and Gewirtz, Stem Cells 18:307-319 (2000); Doudna and Cech, Nature 418:222-228 (2002)) such as the hammerhead ribozymes.

A hammerhead ribozyme is a *cis*-cleaving molecule transformed into a target-specific *trans*-cleaving enzyme with a great potential for applications in biological systems. Preferably, a minimized hammerhead ribozyme is less than 40 nucleotides long and consists of two substrate binding arms and a catalytic domain. Hammerhead ribozymes are known to cleave any NUH triplets (where H is any nucleotide except guanosine) with AUC and GUC triplets being processed most efficiently. Triplets with a cytidine or an adenosine at the second position were reported to be cleavable by hammerhead ribozymes (Kore et al., Nucleic Acids Res. 26:4116-4120 (1998)), although these reactions occurred at lower rates. For applications in cell culture or *in vivo,* ribozymes can either be transcribed from plasmids inside the target cells or they can be administered exogenously. The first approach requires the design of expression cassettes with an RNA polymerase III promoter and stem-loop structures that stabilize the ribozyme (reviewed by Michienzi and Rossi, Methods Enzymol. 341:581-596 (2001)). Due to the fact that RNA is rapidly degraded in biological systems, presynthesized ribozymes have to be protected against nucleolytic attack before they can be used in cell culture or *in vivo* (Beigelman et al., J. Biol. Chem. 270:25702-25708 (1995)). Preferably, a nuclease resistant ribozyme contains five unmodified ribonucleotides, a 2'-*C-*allyl uridine at position 4 and 2'-*O*-methyl RNA at all remaining positions. In addition, the 3' end is protected by an inverted thymidine. A slightly improved version of this ribozyme with four phosphorothioate bonds in one substrate recognition arm and an inverted 3'-3' deoxyabasic can also be used.

Preferably one can use the deoxyribozyme, named'10-23', consisting of a catalytic core of 15 nucleotides and two substrate recognition arms of 6-12 nucleotides on either arm. This deoxyribozyme is highly sequence-specific and can cleave any junction between a purine and a pyrimidine (Joyce, Methods Enzymol. 341:503-517 (2001)).

DNA enzymes with a 3'-3' inverted thymidine can also been used (Santiago et al., Nat. Med. 5:1264-1269 (1999). A DNA enzyme with optimized substrate recognition arms and a partially protected catalytic domain possesses not only increased nuclease resistance but also enhanced catalytic activity.

### III. RNA interference

### III-A. siRNA molecules

In another embodiment, the present invention relates to cell penetrating peptide-small interfering RNA molecules ("siRNA molecules" or "siRNA") conjugates, methods of making said CPP-siRNA molecules conjugates and methods (*e.g*., research and/or therapeutic methods) for using said CPP-siRNA molecules conjugates. A siRNA molecule of the invention is a duplex consisting of a sense strand and complementary antisense strand, the antisense strand having sufficient complementarity to a target mRNA to mediate RNAi. Preferably, the strands are aligned such that there are at least 1, 2, or 3 bases at the end of the strands which do not align *(i.e.,* for which no complementary bases occur in the opposing strand) such that an overhang of 1,2 or 3 residues occurs at one or both ends of the duplex when strands are annealed. Preferably, the siRNA molecule has a length from about 10-50 or more nucleotides, *i.e.,* each strand comprises 10-50 nucleotides (or nucleotide analogs). More preferably, the siRNA molecule has a length from about 15-30 nucleotides. Even more preferably, the siRNA molecule has a length from about 18-25 nucleotides. The siRNA molecules of the invention further have a sequence that is "sufficiently complementary" to a target mRNA sequence to direct target-specific RNA interference (RNAi), as defined herein, *i.e.,* the siRNA has a sequence sufficient to trigger the destruction of the target mRNA by the RNAi machinery or process.

The target RNA cleavage reaction guided by siRNAs is highly sequence specific. In general, siRNA containing a nucleotide sequences identical to a portion of the target gene are preferred for inhibition. However, less than 100% sequence identity between the siRNA and the target gene can be acceptable to practice the present invention. For example, siRNA sequences with insertions, deletions, and single point mutations relative to the target sequence have been found to be effective for inhibition. Moreover, not all positions of a siRNA contribute equally to target recognition. Mismatches in the centre of the siRNA are most critical and essentially abolish target RNA cleavage. Mismatches upstream of the centre or upstream of the cleavage site referencing the antisense strand are tolerated but significantly reduce target RNA cleavage. Mismatches downstream of the centre or cleavage site referencing the antisense strand, preferably located near the 3' end of the antisense strand, *e.g*. 1, 2, 3, 4, 5 or 6 nucleotides from the 3' end of the antisense strand, are tolerated and reduce target RNA cleavage only slightly.

Sequence identity may be determined by sequence comparison and alignment algorithms known in the art. To determine the percent identity of two nucleic acid sequences (or of two amino acid sequences), the sequences are aligned for optimal comparison purposes (*e.g*., gaps can be introduced in the first sequence or second sequence for optimal alignment). The nucleotides (or amino acid residues) at corresponding nucleotide (or amino acid) positions are then compared. When a position in the first sequence is occupied by the same residue as the corresponding position in the second sequence, then the molecules are identical at that position. The percent identity between the two sequences is a function of the number of identical positions shared by the sequences *(i.e.,* % identity = number of identical positions/total number of positions x 100), optionally penalizing the score for the number of gaps introduced and/or length of gaps introduced.

The comparison of sequences and determination of percent identity between two sequences can be accomplished using a mathematical algorithm. In one embodiment, the alignment generated over a certain portion of the sequence aligned having sufficient identity but not over portions having low degree of identity (*i.e*., a local alignment). A preferred, non-limiting example of a local alignment algorithm utilized for the comparison of sequences is the algorithm of Karlin and Altschul (1990) Proc.Natl. Acad. Sci. USA 87: 2264-68, modified as in Karlin and Altschul (1993) Proc.Natl. Acad. Sci. USA 90: 5873-77. Such an algorithm is incorporated into the BLAST programs (version 2.0) of Altschul, et al. (1990) J. Mol. Biol. 215: 403-10. In another embodiment, the alignment is optimized by introducing appropriate gaps and percent identity is determined over the length of the aligned sequences *(i.e.,* a gapped alignment). To obtain gapped alignments for comparison purposes, Gapped BLAST can be utilized as described in Altschul et al., (1997) Nucleic Acids Res. 25 (17): 3389-3402. In another embodiment, the alignment is optimized by introducing appropriate gaps and percent identity is determined over the entire length of the sequences aligned *(i.e.,* a global alignment). A preferred, non-limiting example of a mathematical algorithm utilized for the global comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, aPAM120 weight residue table, a gap length penalty of 12, and a gap penalty of 4 can be used. Greater than 90% sequence identity, *e.g*., 91%, 92%, 93%, 94%, 95%, 96%, 97%, 98%, 99% or even 100% sequence identity, between the siRNA and the portion of the target gene is preferred. Alternatively, the siRNA may be defined functionally as a nucleotide sequence (or oligonucleotide sequence) that is capable of hybridizing with a portion of the target gene transcript (*e.g*., 400 mM NaCl, 40 mM PIPES pH 6.4, 1 mM EDTA,50 C or70 C hybridization for 12-16 hours; followed by washing). Additional preferred hybridization conditions include hybridization at 70 degree C in 1xSSC or 50 degree C in 1xSSC, 50% formamide followed by washing at 70 degree C in 0.3xSSC or hybridization at 70 C in 4xSSC or 50 C in 4xSSC, 50% formamide followed by washing at 67 C in 1xSSC. The hybridization temperature for hybrids anticipated to be less than 50 base pairs in length should be 5-10 degree C less than the melting temperature (Tm) of the hybrid, where Tm is determined according to the following equations. For hybrids less than 18 base pairs in length, Tm (degree C) = 2 (number of A + T bases) + 4 (number of G + C bases). For hybrids between 18 and 49 base pairs in length, Tm (degree C) = 81.5 + 16.6 (log10 [Na+]) + 0.41 (% G+C) - (600/N), where N is the number of bases in the hybrid, and [Na+] is the concentration of sodium ions in the hybridization buffer ([Na+] for 1xSSC = 0. 165 M). Additional examples of stringency conditions for polynucleotide hybridization are provided in Sambrook, J., E. F. Fritsch, and T. Maniatis, 1989, Molecular Cloning : A Laboratory Manual, Cold Spring Harbor Laboratory Press, Cold Spring Harbor, NY, chapters 9 and 11, and Current Protocols in Molecular Biology, 1995, F. M. Ausubel et al.,eds., John Wiley & Sons, Inc., sections 2.10 and 6.3-6.4, incorporated herein by reference. The length of the identical nucleotide sequences may be at least about 10, 12, 15, 17, 20, 22, 25, 27, 30, 32, 35, 37, 40, 42, 45, 47 or 50 bases.

In another aspect, the invention relates to small interfering RNAs (siRNAs) that include a sense strand and an antisense strand, wherein the antisense strand has a sequence sufficiently complementary to a target mRNA sequence to direct target- specific RNA interference (RNAi) and wherein the sense strand and/or antisense strand is modified by the substitution of internal nucleotides with modified nucleotides, such that *in vivo* stability is enhanced as compared to a corresponding unmodified siRNA.

As defined herein, an "internal" nucleotide is one occurring at any position other than the 5' end or 3' end of nucleic acid molecule, polynucleotide or oligonucleotide. An internal nucleotide can be within a single-stranded molecule or within a strand of a duplex or double-stranded molecule. In one embodiment, the sense strand and/or antisense strand is modified by the substitution of at least one internal nucleotide. In another embodiment, the sense strand and/or antisense strand is modified by the substitution of at least 2, 3, 4, 5, 6, 7, 8, 9, 10, 11, 12, 13, 14, 15, 16, 17, 18, 19, 20, 21, 22, 23, 24, 25 or more internal nucleotides. In another embodiment, the sense strand and/or antisense strand is modified by the substitution of at least 5%, 10%, 15%, 20%, 25%, 30%, 35%, 40%, 45%, 50%, 55%, 60%, 65%, 70%, 75%, 80%, 85%, 90%, 95% or more of the internal nucleotides. In yet another embodiment, the sense strand and/or antisense strand is modified by the substitution of all of the internal nucleotides.

In yet another embodiment, the modified nucleotides are present only in the antisense strand. In yet another embodiment, the modified nucleotides are present only in the sense strand. In yet other embodiments, the modified nucleotides are present in both the sense and antisense strand.

Preferred modified nucleotides or nucleotide analogues include sugar- and/or backbone-modified ribonucleotides (*i.e*., include modifications to the phosphate-sugar backbone). For example, the phosphodiester linkages of natural RNA may be modified to include at least one of a nitrogen or sulfur heteroatom. In preferred backbone-modified ribonucleotides the phosphoester group connecting to adjacent ribonucleotides is replaced by a modified group, *e.g*., of phosphothioate group. In preferred sugar-modified ribonucleotides, the 2' moiety is a group selected from H, OR, R, halo, SH, SR, NH2, NHR, NR2 or ON, wherein R isC1-C6 alkyl, alkenyl or alkynyl and halo is F, Cl, Br or I.

Preferred are 2'-fluoro, 2'-amino and/or 2'-thio modifications. Particularly preferred modifications include 2'-fluoro-cytidine, 2'-fluoro-uridine, 2'-fluoro-adenosine, 2'-fluoroguanosine, 2'-amino-cytidine, 2'-amino-uridine, 2'-amino-adenosine, 2'-amino-guanosine, 2,6-diaminopurine, 4-thio-uridine; and/or 5-amino-allyl-uridine. Additional exemplary modifications include 5-bromo-uridine, 5-iodo-uridine, 5-methyl-cytidine, ribo-thymidine, 2-aminopurine, 2'-amino-butyryl-pyrene-uridine, 5-fluoro-cytidine, and 5-fluoro-uridine. 2'-deoxy-nucleotides can be used within modified siRNAs of this invention, but are preferably included within the sense strand of the siRNA duplex. Additional modified residues have been described in the art and are commercially available including, deoxy-abasic, inosine, N3-methyl- uridine, N6, N6-dimethyl-adenosine, pseudouridine, purine ribonucleoside and ribavirin.

Modification of the linkage between nucleotides or nucleotide analogs is also preferred, *e.g*., substitution of phosphorothioate linkages for phosphodiester linkages.

Also possible are nucleobase-modified ribonucleotides, *i.e.,* ribonucleotides, containing at least one non-naturally occurring nucleobase instead of a naturally occurring nucleobase. Bases may be modified to block the activity of adenosine deaminase. Exemplary modified nucleobases include, but are not limited to, uridine and/or cytidine modified at the 5-position, *e.g*., 5-(2-amino)propyl uridine, 5-bromo uridine; adenosine and/or guanosines modified at the 8 position, *e.g*., 8-bromo guanosine; deaza nucleotides, *e.g*., 7-deazaadenosine; O-and N-alkylated nucleotides, *e.g*., N6-methyl adenosine are suitable.

It should be noted that all modifications described herein may be combined. In a preferred embodiment, 2'-fluoro modified ribonucleotides and 2'-deoxy ribonucleotides are combined and both are present within the antisense strand.

Preferably, a siRNA molecule of the invention will have a three-dimensional structure resembling A-form RNA helix. More preferably, a siRNA molecule of the invention will have an antisense strand which is capable of adopting an A-form helix when in association with a target RNA (*e.g.*, an mRNA). For this reason, 2'-fluoro- modified nucleotides are preferred, as siRNA made with such modified nucleotides adopts an A-form helix confirmation. In particular, it is important that a siRNA be capable of adopting an A-form helix in the portion complementary to the target cleavage site as it has been discovered that the major groove formed by the A-form helix at the cleavage site, and not the RNA itself, is an essential determinant of RNAi.

Even more preferably, a siRNA molecule will exhibit increased stability (*i.e.,* resistance to cellular nucleases) as compared to an unmodified siRNA molecule.

### IIIB. siRNA Derivatives

In another embodiment, the present invention relates to cell penetrating peptide-small interfering RNA derivative conjugates.

A siRNA derivative is a siRNA having at least one of the following which is not a feature of siRNA: a label at the 3' terminus (*e.g*., biotin or a fluorescent molecule), the 3' terminus is blocked, the 3' terminus has a covalently linked group or compound (*e.g*., a nanoparticle), the siRNA derivative does not form a perfect A-form helix, but the antisense strand of the siRNA derivative duplex does form an A-form helix with target RNA, or the siRNA derivative is crosslinked (*e.g*., by psoralen). Methods of synthesizing RNAs and modifying RNAs are known in the art (*e.g.*, Hwang et al., 1999, Proc. Nat. Acad. Sci. USA 96: 12997-13002; and Huq and Rana, 1997, Biochem. 36: 12592-12599).

Certain chemical modifications confer useful properties to siRNA. For example, increased stability compared to an unmodified siRNA or a label that can be used, *e.g*., to trace the siRNA, to purify a siRNA, or to purify the siRNA and cellular components with which it is associated.

SiRNA derivatives containing certain functional groups such as biotin are useful for affinity purification of proteins and molecular complexes involved in the RNAi mechanism.

Crosslinked siRNA derivatives: Some embodiments include the use of siRNAs that contain one or more crosslinks between nucleic acids in the complementary strands of the siRNA. Crosslinks can be introduced into a siRNA using methods known in the art. In addition to crosslinking using psoralen (*e.g*., Wang et al., 1996, J. Biol. Chem. 271: 16995-16998) other methods of crosslinking can be used. In some embodiments, photocrosslinks are made containing thiouracil (*e.g*., 4-thiouridine) or thioguanosine bases. In other embodiments, -SH linkers can be added to the bases or sugar backbones, which are used to make S-S crosslinks. In some cases, sugar backbones or amino groups at the C5 position of U, C can be labelled with benzophenone and other photo crosslinkers or with chemical crosslinkers. Methods of making such crosslinks are known in the art (*e.g*., Wang and Rana, 1998, Biochem. 37: 4235-4243; BioMosaics, Inc., Burlington, VT). In general, the stability in a cell or a cell-free system of a crosslinked siRNA derivative is greater than that of the corresponding siRNA. In some cases, the crosslinked siRNA derivative has less activity than the corresponding siRNA. The ability of a crosslinked siRNA to inhibit expression of a target sequence can be assayed using methods known in the art for testing the activity of a siRNA or by methods disclosed herein such as a dual fluorescence reporter gene assay.

In general, a siRNA derivative that is crosslinked contains one crosslink between two nucleotides of a dsRNA sequence. In some embodiments, there are two or more crosslinks. Crosslinks are generally located near the 3' terminus of the antisense strand, *e.g*., within about 10 nucleotides of the 3' terminus of the antisense strand, and generally within about 2-7 nucleotides of the 3' terminus of the antisense strand. A crosslink is to be distinguished from ligation that joins the ends of the two strands of a siRNA. A mixture of crosslinked siRNA derivatives that contains some molecules crosslinked at loci near the middle of the siRNA or near the 5' terminus of the antisense strand can also be useful. Such mixtures can have less activity than mixture of siRNA derivative that is crosslinked exclusively near the 3' terminus, but retain sufficient activity to affect expression of a targeted sequence.

3' modifications of siRNA: Molecules that are used for affinity purification or as detectable tags can be covalently linked to the 3' terminus of an RNAi to create a siRNA derivative. Such RNAi derivatives are useful, *e.g*., for assaying a siRNA by transfecting a cell with a siRNA derivative of the siRNA containing a detectable tag at the 3' end and detecting the tag using methods known in the art. Examples of such tags that can be used for detection or affinity purification of derivative siRNAs include biotin.

Methods that can be used to modify a siRNA are known in the art. For example, crosslinkers can be attached using amino-allyl coupling methods, *e.g.,* isothiocyanate, N-hydroxysuccinimide (NHS) esters (Amersham Biosciences Corp., Piscataway, NJ). Crosslinkers can be attached to amino-allyl uridine or amino groups at sugars using similar chemistry.

In some embodiments, photocrosslinkers (*e.g*., thiouracil, thioguanosine, psoralens, benzophenones) are attached at 3' terminus of a siRNA to create a siRNA derivative. Methods of synthesizing such modifications are known in the art. Such a siRNA derivative can be crosslinked to the target cellular machinery *in vitro* and *in vivo.*

In other embodiments, a dye can be linked to 3' termini of a siRNA. Such dyes include those that are useful for energy transfer and functional assays, *e.g*., of helicase activity. For example, a fluorescent donor dye such as isothiocyanatefluorescein can be attached to the 3' end of the antisense strand of a siRNA. An acceptor dye (*e.g*., isothiocyanate rhodamine) can be attached to the 5' end. RNA-containing amino groups at the 3' or 5' end can be obtained from commercial sources or appropriate dyes can be purchased and the molecules synthesized (Integrated DNA Technologies, Coralville, Iowa). Such a modified siRNA can be incubated with RISC complex that contains helicase. Fluorescence resonance energy transfer (FRET) signals will be altered when the RNA helix of the modified siRNA is unwound.

Modification of the 3' end can also include attachment of photocleavable compounds such as biotin. A photocleavable biotin can be synthesized according to the method depicted in PCT patent application WO 04/02912.

### III.C. Production

RNA may be produced enzymatically or by partial/total organic synthesis, any modified ribonucleotide can be introduced by *in vitro* enzymatic or organic synthesis. In one embodiment, a siRNA is prepared chemically. Methods of synthesizing RNA molecules are known in the art, in particular, the chemical synthesis methods as described in Verma and Eckstein (1998) Annul Rev. Biochem. 67: 99-134. In another embodiment, a siRNA is prepared enzymatically. For example, a ds-siRNA can be prepared by enzymatic processing of a long dsRNA having sufficient complementarity to the desired target mRNA. Processing of long dsRNA can be accomplished *in vitro,* for example, using appropriate cellular lysates and ds-siRNAs can be subsequently purified by gel electrophoresis or gel filtration. ds-siRNA can then be denatured according to art-recognized methodologies. In an exemplary embodiment, RNA can be purified from a mixture by extraction with a solvent or resin, precipitation, electrophoresis, chromatography, or a combination thereof. Alternatively, the RNA may be used with no or a minimum of purification to avoid losses due to sample processing.

Alternatively, the single-stranded RNAs can also be prepared by enzymatic transcription from synthetic DNA templates or from DNA plasmids isolated from recombinant bacteria. Typically, phage RNA polymerases are used such as T7, T3 or SP6 RNA polymerase (Milligan and Uhlenbeck (1989) Methods Enzymol. 180: 51-62). The RNA may be dried for storage or dissolved in an aqueous solution. The solution may contain buffers or salts to inhibit annealing, and/or promote stabilization of the single strands.

In one embodiment, siRNAs are synthesized either *in vivo, in situ,* or *in vitro.* Endogenous RNA polymerase of the cell may mediate transcription *in vivo* or *in situ,* or cloned RNA polymerase can be used for transcription *in vivo* or *in vitro.* For transcription from a transgene *in vivo* or an expression construct, a regulatory region (*e.g*., promoter, enhancer, silencer, splice donor and acceptor, polyadenylation) may be used to transcribe the siRNA. Inhibition may be targeted by specific transcription in an organ, tissue, or cell type; stimulation of an environmental condition (*e.g*., infection, stress, temperature, chemical inducers); and/or engineering transcription at a developmental stage or age. A transgenic organism that expresses siRNA from a recombinant construct may be produced by introducing the construct into a zygote, an embryonic stem cell, or another multipotent cell derived from the appropriate organism.

### III.D. Targets

In one embodiment, the target mRNA encodes the amino acid sequence of a cellular protein (*e.g*., a nuclear, cytoplasmic, transmembrane, or membrane-associated protein). In another embodiment, the target mRNA encodes the amino acid sequence of an extracellular protein (*e.g*., an extracellular matrix protein or secreted protein). As used herein, the phrase "encodes the amino acid sequence" of a protein means that the mRNA sequence is translated into the amino acid sequence according to the rules of the genetic code. The following classes of proteins are listed for illustrative purposes: developmental proteins (*e.g*., adhesion molecules, cyclin kinase inhibitors, Wnt family members, Pax family members, Winged helix family members, Hox family members, cytokines/lymphokines and their receptors, growth/differentiation factors and their receptors, neurotransmitters and their receptors); oncogene-encoded proteins (*e.g.*, ABLI, BCLI, BCL2, BCL6, CBFA2, CBL, CSFIR, ERBA, ERBB, EBRB2, ETSI, ETSI, ETV6, FGR, FOS, FYN, HCR, HRAS, JUN, KRAS, LCK, LYN, MDM2, MLL, MYB, MYC, MYCLI, MYCN, NRAS, PIM I, PML, RET, SRC, TALI, TCL3, and YES); tumor suppressor proteins (*e.g*., APC,BRCA1, BRCA2, MADH4, MCC, NFI, NF2, RBI, TP53, and WTI); transcription factors; house-keeping proteins; cytoskeleton-related proteins; receptor-related proteins; cytokines; angiogenic proteins; growth factor proteins; and enzymes (*e.g*., ACC synthases and oxidases, ACP desaturases and hydroxylases, ADP- glucose pyrophorylases, ATPases, alcohol dehydrogenases, amylases, amyloglucosidases, catalases, cellulases, chalcone synthases, chitinases, cyclooxygenases, decarboxylases, dextriinases, DNA and RNA polymerases, galactosidases, glucanases, glucose oxidases, granule-bound starch synthases, GTPases, helicases, hernicellulases, integrases,inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

In another aspect of the invention, the target mRNA molecule of the invention encodes the amino acid sequence of a protein associated with a pathological condition. For example, the protein may be a pathogen-associated protein (*e.g*., a viral protein involved in immunosuppression of the host, replication of the pathogen, transmission of the pathogen, or maintenance of the infection), or a host protein which facilitates entry of the pathogen into the host, drug metabolism by the pathogen or host, replication or integration of the pathogen's genome, establishment or spread of infection in the host, or assembly of the next generation of pathogen. Alternatively, the protein may be a tumor-associated protein or an autoimmune disease-associated protein.

In one embodiment, the target mRNA molecule encodes the amino acid sequence of an endogenous protein *(i.e.,* a protein present in the genome of a cell or organism). In another embodiment, the target mRNA molecule encodes the amino acid sequence of a heterologous protein expressed in a recombinant cell or a genetically altered organism (*e.g*. altered by transgenic or knockout technologies). In another embodiment, the target mRNA molecule encoded the amino acid sequence of a protein encoded by a transgene (*i.e*., a gene construct inserted at an ectopic site in the genome of the cell). In yet another embodiment, the target mRNA molecule of the invention encodes the amino acid sequence of a protein encoded by a pathogen genome which is capable of infecting a cell or an organism from which the cell is derived.

### III.E. Targeting VEGF

Angiogenesis has been specifically linked to increased growth and metastatic potential in human tumors (Ferrara, Semin. Oncol. 29: 10-45 (2002)). Although numerous growth factors are involved, "vascular endothelial growth factor" ("VEGF") has been shown to play a pivotal role in tumor angiogenesis (Fernando et al., Semin. Oncol. 30:39-506 (2003)). Binding of VEGF to its receptors induces mitogenesis and chemotaxis of normal endothelial cells and increases vascular permeability, all of which contribute to new vessel formation and tumor growth (Yancopoulos et al., Nature 407:242-87 (2000)). VEGF also contributes to neovascularization by mobilizing bone marrow-derived endothelial progenitor cells ( Asahara et al., EMBO J. 18:3964-728 (1999)). To date, six alternatively spliced isoforms of human VEGF have been identified (VEGF)₁₂₁, VEGF₁₄₅, VEGF₁₆₅, VEGF₁₈₃, VEGF₁₈₉, and VEGF₂₀₆ ; Ferrara et al., Endocr. Rev. 18:4-25 (1997)). Increased levels of VEGF expression have been found in most human tumors, including those of the lung, gastrointestinal tract, kidney, thyroid, bladder, ovary and cervix (Ferrara, J. Mol. Med. 77:527-4310 (1999)).

In another aspect, the invention features methods of treating a subject having a disorder characterized by unwanted cellular proliferation, *e.g*., cancers, *e*.*g.,* carcinomas, sarcomas, metastatic disorders and hematopoietic neoplastic disorders (*e.g*., leukemias), age related macular degenerative disorders or proliferative skin disorders, *e.g*., psoriasis, by administering to the subject an amount of a conjugate of the invention, *e.g.,* a therapeutic composition, of the invention, effective to inhibit VEGF expression, secretion or activity. As used herein, inhibiting VEGF expression or activity refers to a reduction in the expression or activity of VEGF, *e.g*., by 10%, 20%, 30%, 40%, 50%, 60%, 70%, 80%, 90%, or 100%.

VEGF nucleic acid targets: In one aspect, the invention features compositions (*e.g*., siRNAs-, siRNA derivatives-, modified siRNAs-CPP conjugates) that are targeted to a VEGF RNA.

The mRNA sequence of VEGF can be any ortholog of VEGF, such as sequences substantially identical to the human VEGF, including but not limited to GenBank Accession No: NM_001025367, NM_001025368, NM_001025369 or NM_001025370.

### siRNA molecules:

The siRNAs of the invention include dsRNA molecules comprising 16-30 nucleotides in each strand, wherein one of the strands is substantially identical, *e.g*., at least 80% identical, to a target region in the mRNA of VEGF, and the other strand is identical or substantially identical to the first strand.

The siRNAs, siRNA derivatives, modified siRNAs of the compositions can be chemically synthesized, or can be transcribed *in vitro* from a DNA template, or *in vivo* from, *e.g*., shRNA. The dsRNA molecules can be designed using any method known in the art.

The siRNA molecule include both unmodified VEGF siRNAs and modified VEGF siRNAs as known in the art, such as crosslinked siRNA derivatives. Crosslinking can be employed to alter the pharmacokinetics of the siRNA, siRNA derivative, or modified siRNA.

The dsRNA molecules of the present invention can comprise the following sequence as one of their strands, and the corresponding sequences of allelic variants thereof:
Sense strand: 5' AUG UGA AUG CAG ACC AAA GAA-dTsdT (Filleur et al., Cancer Res. 63:3919-22 (2003))

The above sequence (*e.g*., sense sequence) corresponds to targeted portions of its target mRNAs, as described herein. Reverse complementary sequences (*e.g*., antisense sequences) can be generated according to art recognized principles. dsRNA molecules of the present invention preferably comprise one sense sequence or strand and one respective antisense sequence or strand.

Moreover, because RNAi is believed to progress via at least one single stranded RNA intermediate, the skilled artisan will appreciate that ss-siRNAs (*e.g*., the antisense strand of a ds-siRNA) can also be designed as described herein and utilized according to the claimed methodologies.

### III.F. Methods of use of the CPP-siRNA conjugates

The CPP-siRNA conjugates of the invention can be introduced in a cell or organism by any method known in the art.

In mammals, siRNA molecules are rapidly excreted by the kidney. Advantageously, the CPP-siRNA of the invention can decrease the siRNA plasma clearance.

In another embodiment CPP-siRNA conjugate may be introduced along with components that perform one or more of the following activities: enhance conjugate uptake by the cell, inhibit annealing of single strands, stabilize the single strands, or otherwise increase inhibition of the target gene.

The CPP-siRNA conjugates of the invention can be introduced extracellularly into a cavity (*e*.*g*. intraperitoneally) interstitial space, into the circulation of an organism, introduced orally, or may be introduced by bathing a cell, organ or organism in a solution containing the conjugate. Vascular or extravascular circulation, the blood or lymph system, and the cerebrospinal fluid are sites where the CPP-siRNA conjugate may be introduced.

The cell with the target gene may be derived from or be contained in any organism. The organism may a plant, animal, protozoan, bacterium, virus, or fungus. The animal may be a vertebrate or invertebrate. Examples of vertebrate animals include fish, mammal, cattle, goat, pig, sheep, rodent, hamster, mouse, rat, primate, and human.

The cell having the target gene may be from the germ line or somatic, totipotent or pluripotent, dividing or non-dividing, parenchyma or epithelium, immortalized or transformed, or the like. The cell may be a stem cell or a differentiated cell. Cell types that are differentiated include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, epithelia, displasic tissues and cells of the endocrine or exocrine glands.

Depending on the particular target gene and the dose of double stranded RNA material delivered, this process may provide partial or complete loss of function for the target gene. A reduction or loss of gene expression in at least 30%, 40%, 50%, 60%, 70%, 80%, 90%, 95% or 99% or more of targeted cells is exemplary. Inhibition of gene expression refers to the absence (or observable decrease) in the level of protein and/or mRNA product from a target gene. Specificity refers to the ability to inhibit the target gene without manifest effects on other genes of the cell. The consequences of inhibition can be confirmed by examination of the outward properties of the cell or organism (as presented below in the examples) or by biochemical techniques such as RNA solution hybridization, nuclease protection, Polymerase Chain Reaction (PCR), Northern hybridization, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS).

As an example, the efficiency of inhibition may be determined by assessing the amount of gene product in the cell or excreted by the cell; mRNA may be detected with a hybridization probe having a nucleotide sequence outside the region used for the inhibitory double-stranded RNA, or translated polypeptide may be detected with an antibody raised against the polypeptide sequence of that region.

The CPP-siRNA conjugate may be introduced in an amount which allows delivery of at least one siRNA copy per cell. Higher doses (*e.g*., at least 5, 10, 100, 500 or 1000 copies per cell) of material may yield more effective inhibition; lower doses may also be useful for specific applications.

The present invention has as another object a process for preparing a compound having the formula P-L-N, wherein P is a cell penetrating peptide, L is PEG-based linker linking P and N together and N is a nucleic acid, preferably an oligonucleotide and more preferably a siRNA.

In a first embodiment, the process comprises the steps of coupling (*i.e*. conjugating) the nucleic acid (*e.g*. a siRNA) with the PEG-based linker in an appropriate buffer (preferably a phosphate buffer whose pH is between 6.5 and 8.5) and then coupling (*i. e*. conjugating) the PEG-based linker-nucleic conjugate acid with the cell penetrating peptide in an appropriate buffer (preferably a phosphate buffer whose pH is between 6.5 and 8.5).

In a second embodiment, the process comprises the steps of coupling (*i.e*. conjugating) the cell penetrating peptide with the PEG-based linker in an appropriate buffer (preferably a phosphate buffer whose pH is between 6.5 and 8.5) and then coupling (*i.e*. conjugating) the PEG-based linker-cell penetrating peptide conjugate with the nucleic acid (*e.g*. a siRNA) in an appropriate buffer (preferably a phosphate buffer whose pH is between 6.5 and 8.5).

Preferably, to permit the coupling of the nucleic acid comprises with the PEG-based linker, the nucleic acid comprises at least one -NHS or -SH moiety, and the PEG-based linker comprises at least one -S-S-pyridine, -N-hydroxysuccinimide, -COOH or -SH moiety.

Preferably also, to permit the coupling of the PEG-based linker with the cell penetrating peptide, the cell penetrating peptide comprises at least one -SH or -NH₂ moity, and the PEG-based linker comprises at least one maleimide, -N-hydroxysuccinimide, -NH₂, - COOH or -NH moiety.

The conjugation of PEG onto peptides or proteins is known from one skilled in the art; see for example, PCT patent application No WO 90/13540 which describes PEG that is converted into its N-succinimide carbonate derivative.

Synthesis of PEG-nucleic acids conjugate is also well known from one skilled in the art; see for example Jäschke et al., Nucleic. Acids Research, 22:4810-7 (1994) or Jeong et al., J Control Release. 93:183-91 (2003).

Detailed processes are described in the examples.

The present invention also provides for both prophylactic and therapeutic methods of treating a subject at risk of (or susceptible to) a disorder or having a disorder associated with aberrant or unwanted target gene expression or activity. "Treatment", or "treating" as used herein, is defined as the application or administration of a CPP-nucleic acid conjugate of the present invention (*e*.*g*., CPP-siRNA) to a patient, or application or administration of a therapeutic agent to an isolated tissue or cell line from a patient, who has a disease or disorder, a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease. For siRNA, the treatment can include administering siRNAs to one or more target sites. The mixture of different siRNAs-CPP conjugates can be administered together or sequentially, and the mixture can be varied over time.

With regards to both prophylactic and therapeutic methods of treatment, such treatments may be specifically tailored or modified, based on knowledge obtained from the field of pharmacogenomics. The terms "pharmacogenomic(s)" and "pharmacogenetic(s)" are used herein interchangeably and, refer to the application of genomics technologies such as gene sequencing, statistical genetics, and gene expression analysis to drugs in clinical development and on the market. More specifically, the term refers the study of how a patient's genes determine his or her response to a drug (*e.g*., a patient's "drug response phenotype", or "drug response genotype"). Thus, another aspect of the invention provides methods for tailoring an individual's prophylactic or therapeutic treatment with either the target gene molecules of the present invention or target gene modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

### Prophylactic Methods:

In one aspect, the invention provides a method for preventing in a subject, a disease or condition associated with an aberrant or unwanted target gene expression or activity, by administering to the subject a CPP-nucleic acid conjugate of the present invention (*e.g*., CPP-siRNA). Subjects at risk for a disease which is caused or contributed to by aberrant or unwanted target gene expression or activity can be identified by, for example, any or a combination of diagnostic or prognostic assays as described herein.

Administration of a prophylactic agent can occur prior to the manifestation of symptoms characteristic of the target gene aberrancy, such that a disease or disorder is prevented or, alternatively, delayed in its progression. Depending on the type of target gene aberrancy, for example, a target gene, target gene agonist or target gene antagonist agent can be used for treating the subject. The appropriate agent can be determined by one skilled in the art based on screening assays.

### Therapeutic Methods:

Another aspect of the invention pertains to methods of modulating target gene expression, protein expression or activity for therapeutic purposes. Accordingly, in an exemplary embodiment, the modulatory method of the invention involves contacting a cell capable of expressing target gene with a CPP-nucleic acid conjugate of the present invention (*e.g*., CPP-siRNA) that is specific for the target gene or protein (*e.g*. if the nucleic acid is a siRNA, then the siRNA is specific for the mRNA encoded by said gene or specifying the amino acid sequence of said protein) such that expression or one or more of the activities of target protein is modulated. These modulatory methods can be performed *in vitro (e.g.,* by culturing the cell with the conjugate) or, alternatively, *in vivo (e.g.,* by administering the conjugate to a subject). As such, the present invention provides methods of treating an individual afflicted with a disease or disorder characterized by aberrant or unwanted expression or activity of a target gene polypeptide or nucleic acid molecule. Inhibition of target gene activity is desirable in situations in which target gene is abnormally unregulated and/or in which decreased target gene activity is likely to have a beneficial effect.

### Pharmacogenomics:

The CPP-nucleic acid conjugate of the present invention (e.g., CPP-siRNA) can be administered to individuals to treat (prophylactically or therapeutically) disorders associated with aberrant or unwanted target gene activity. In conjunction with such treatment, "pharmacogenomics" or "pharmacogenetics" (both terms are used interchangeably) *(i.e.,* the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a therapeutic agent as well as tailoring the dosage and/or therapeutic regimen of treatment with a therapeutic agent. Pharmacogenomics deals with clinically significant hereditary variations in the response to drugs due to altered drug disposition and abnormal action in affected persons. See, for example, Eichelbaum et al. Clin. Exp.Pharmacol. Physiol. 23:983-985 (1996) and Linder et al. Clin. Chem. 43: 254-266 (1997). In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

### Disease Indications:

The compositions of the invention can act as novel therapeutic agents for controlling one or more of cellular proliferative and/or differentiative disorders, disorders associated with bone metabolism, immune disorders, hematopoietic disorders, cardiovascular disorders, liver disorders, kidney disorders, muscular disorders, haematological disorders, viral diseases, pain or neurological disorders, or metabolic disorders. Examples of cellular proliferative and/or differentiative disorders include cancer, *e.g*., carcinoma, sarcoma, metastatic disorders or hematopoietic neoplastic disorders, *e.g*., leukemias. A metastatic tumor can arise from a multitude of primary tumor types, including but not limited to those of prostate, colon, lung, breast and liver origin.

As used herein, the terms "cancer", "hyperproliferative", and "neoplastic" refer to cells having the capacity for autonomous growth, *i.e.,* an abnormal state or condition characterized by rapidly proliferating cell growth. Hyperproliferative and neoplastic disease states may be categorized as pathologic, *i.e*., characterizing or constituting a disease state, or may be categorized as non-pathologic, *i.e*., a deviation from normal but not associated with a disease state. The term is meant to include all types of cancerous growths or oncogenic processes, metastatic tissues or malignantly transformed cells, tissues, or organs, irrespective of histopathologic type or stage of invasiveness.

"Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, kidney, prostate, breast, head and neck, colon and ovary. The term also includes carcinosarcomas, *e.g*., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

Additional examples of proliferative disorders include hematopoietic neoplastic disorders. As used herein, the term "hematopoietic neoplastic disorders" includes diseases involving hyperplastic/neoplastic cells of hematopoietic origin, *e.g*., arising from myeloid, lymphoid or erythroid lineages, or precursor cells thereof. Preferably, the diseases arise from poorly differentiated acute leukemias, *e.g*., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) CritRev. in Oncol. 1Hemotol. 11: 267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

In general, the compositions of the invention are designed to target genes associated with particular disorders. Examples of such genes associated with proliferative disorders that can be targeted include activated ras, p53, BRCA-1, and BRCA-2. Other specific genes that can be targeted are those associated with amyotrophic lateral sclerosis (ALS; *e.g.,* superoxidedismutase-1(SOD1)) ; Huntington's disease *(e.g.,* huntingtin), Parkinson's disease (parkin), and genes associated with autosomal dominant disorders.

The compositions of the invention can be used to treat a variety of immune disorders, in particular those associated with overexpression of a gene or expression of a mutant gene. Examples of hematopoietic disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmunethyroiditis, dermatitis (including atopic dermatitis and eczematous dermatitis), psoriasis, Sjgren's Syndrome, Crohn's disease, aphthous ulcer, iritis, conjunctivitis, keratoconjunctivitis, ulcerative colitis, asthma, allergic asthma, cutaneous lupus erythematosus,scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves'disease, sarcoidosis, primarybiliary cirrhosis, uveitis posterior, and interstitial lung fibrosis), graft-versus- host disease, cases of transplantation, and allergy such as, atopic allergy.

Examples of disorders involving the heart or "cardiovascular disorder" include, but are not limited to, a disease, disorder, or state involving the cardiovascular system, *e.g*., the heart, the blood vessels, and/or the blood. A cardiovascular disorder can be caused by an imbalance in arterial pressure, a malfunction of the heart, or an occlusion of a blood vessel, e. g., by a thrombus. Examples of such disorders include hypertension, atherosclerosis, coronary artery spasm, congestive heart failure, coronary artery disease, valvular disease, arrhythmias, and cardiomyopathies.

Disorders which may be treated by methods described herein include, but are not limited to, disorders associated with an accumulation in the liver of fibrous tissue, such as that resulting from an imbalance between production and degradation of the extracellular matrix accompanied by the collapse and condensation of preexisting fibers.

Additionally, molecules of the invention can be used to treat viral diseases, including but not limited to hepatitis B, hepatitis C, herpes simplex virus (HSV), HIV- AIDS, poliovirus, and smallpox virus. Molecules of the invention are engineered as described herein to target expressed sequences of a virus, thus ameliorating viral activity and replication. The molecules can be used in the treatment and/or diagnosis of viral infected tissue. Also, such molecules can be used in the treatment of virus-associated carcinoma, such as hepatocellular cancer.

The invention pertains to uses of the above-described CPP-nucleic acid conjugates for therapeutic treatments as described supra. Thus, the scope of the invention extends to the use of a CPP-nucleic acid conjugates of the invention for the manufacture of a medicament (or pharmaceutical) for treating or preventing a disorder as described supra. Accordingly, the CPP-nucleic acid conjugates of the present invention can be incorporated into compositions, preferably pharmaceutical compositions, suitable for administration. Such compositions typically comprise at least one conjugate according to the present invention or a mixture of conjugates and optionally, a pharmaceutically acceptable carrier. As used herein "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A composition of the invention, preferably pharmaceutical composition, is formulated to be compatible with its intended route of administration. Examples of routes of administration include parenteral, *e*.*g*., intravenous, intradermal, subcutaneous, intraperitoneal, intramuscular, oral (*e.g.*, inhalation), transdermal (topical), transmucosal, intraocular, and intratumoral administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, CremophorELTM (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems.

Biodegradable, biocompatible polymers can be used, such as ethylene vinyl acetate, polyanhydrides, polyglycolic acid, collagen, polyorthoesters, and polylactic acid.

Methods for preparation of such formulations will be apparent to those skilled in the art. The materials can also be obtained commercially from Alza Corporation for example and can also be used as pharmaceutically acceptable carriers. These can be prepared according to methods known to those skilled in the art, for example, as described in U. S. Patent No. 4,522, 811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, *e.g*., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. Although compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the EC50 *(i.e.,* the concentration of the test compound which achieves a half-maximal response) as determined in cell culture. Such information can be used to more accurately determine useful doses in humans. Levels in plasma may be measured, for example, by high performance liquid chromatography.

A therapeutically effective amount *(i.e.,* an effective dosage) of a composition containing a CPP-nucleic-acid conjugate of the invention (*e.g*., a CPP-siRNA) is easily determined by one skilled in the art. For example for CPP-siRNA conjugate, a therapeutically effective amount is an amount that inhibits expression of the polypeptide encoded by the target gene by at least 10 percent. Higher percentages of inhibition, *e.g*., 45, 50, 55, 60, 65, 70, 75, 80, 85, 90, 95 percent or higher may be preferred in certain embodiments. Exemplary doses include milligram or microgram amounts of the conjugate per kilogram or m² of subject or sample weight *(e.g*., about 1 microgram per kilogram or m² to about 500 milligrams per kilogram or m², about 100 micrograms per kilogram or m² to about 5 milligrams per kilogram or m², or about 1 microgram per kilogram to about 50 micrograms per kilogram or m². The compositions can be administered at least once per week for between about 1 to 10 weeks..

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments.

It is furthermore understood that appropriate doses of a composition depend upon the potency of composition with respect to the expression or activity to be modulated.

When one or more of these conjugates of the invention is to be administered to an animal (*e.g*., a human) to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The pharmaceutical compositions can be included in a container, pack, or dispenser together with instructions for administration.

### BRIEF DESCRIPTION OF THE DRAWINGS

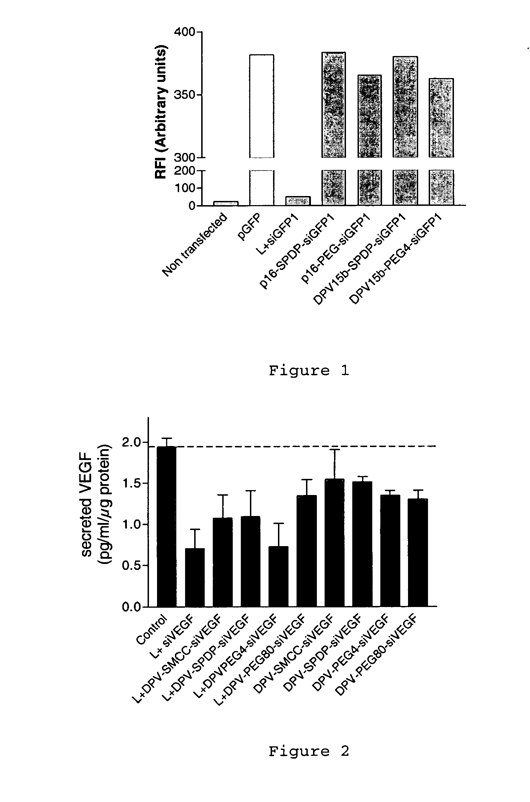

Figure 1 shows the efficiency studies (by measuring RFI (Relative Fluorescence Intensity)) comparing two CPPs, penetratin (long version, denoted p16) and DPV15b linked to siRNA eGFP (siGFP1) via a SPDP or PEG₄-based linker were performed in eGFP-transient transfected PC-3 cells. Activity of CPP-siRNA was assessed 48 hours after continuous incubation of CPP-siRNA molecules. "L" means transfection with Lipofectamine^{™} 2000
Figure 2 shows the effect of linker on VEGF secretion in MDA-MB-231 cells. MDA-MB-231 cells were treated with the indicated DPV15b-linker-siVEGF (SMCC, SPDP, PEG₄-based linker and PEG₈₀-based linker) for 20 h. Conditionned medium is analysed by ELISA. Cells were lysed and proteins were quantified by Bradford assay for internal standardisationThen the cells were lysed and the supernatant analyzed for VEGF secretion as described under Materials and methods. For control, the supernatant of MDA-MB-231 untreated was analysed after 20h of incubation. Data are presented as means ± SE, n = 3. "L" means transfection with Lipofectamine^{™} 2000.

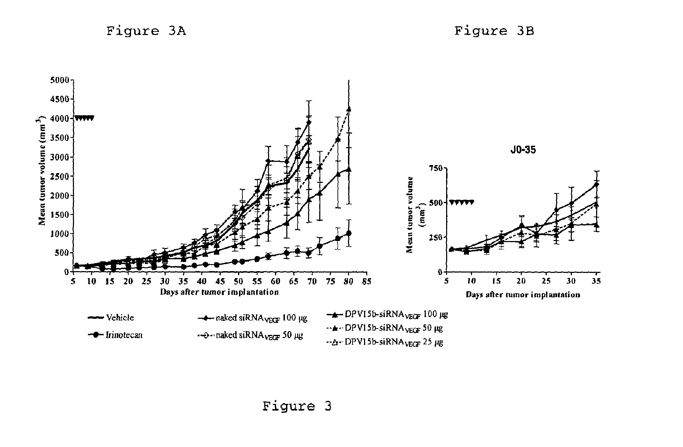

Figure 3 shows the *in vivo* efficacy study of DPV-(PEG₄-based linker)-siRNA_{VEGF} conjugate and naked siRNA_{VEGF} in MDA-MB-231 human breast carcinoma model. Treatments were administered by the *i.v.* route on days 6 to 10 (Q1D5 administration schedule, see arrowheads), the mice receiving a constant volume (10 µL/g) of either saline (-) or of the different dosing solutions: DPV15b-siRNA_{VEGF} at 100 (-▲-), 50 (--▲--) or 25 (--Δ--) µg eq siRNA/mouse, siRNA_{VEGF} at 100 (-◆-) or 50 (--◇--) µg/mouse or Irinotecan at 48.15 µmol/kg (-•-). Results represent the mean tumor volume evolution from day 0-80 (Figure 3A) or day 0-35 (Figure 3B).

### EXAMPLES

Other advantages and characteristics of the invention will appear from the following examples which refer to the above figures. The examples are given to illustrate the invention but not to limit the scope of the claims.

### I- Conjugation Protocol

### I-1 Method for the preparation of a conjugate represented by the structure of the general formula P-L-N, wherein P is a cell penetrating peptide, N is a siRNA, and L is a polyethylene glycol (PEG)-based linker linking P and N together: single strand approach

1) dissolve the 5' amino C3 sense siRNA strand *(i.e.* NH₂-(CH₂)₃-phosphate moiety anchored on the free 5' moiety of the sense strand) in sterile water (2mM),
2) dilute the 5' amino C3 sense strand with phosphate buffer pH 8.2 (50 mM NaH₂PO₄/Na₂HPO4 50 mM, NaCl O.15M) to a final siRNA concentration of 2 mg.mL⁻¹,
3) after addition of 400 eq. of PEG-based linker (solubilized 10 mg.mL⁻¹ in N,N-dimethylformamide) incubate the reaction mixture 90 min. at room temperature (about 20 °C),
4) dissolve the antisense siRNA in sterile water (2mM) and hybridise 1 eq. to the activated sense siRNA strand by incubation 15 min at 80°C, followed by slow cooling to room temperature (about 20°C) to allow siRNA duplex formation,
5) precipitate the duplexes by the addition of 0.3 vol. of NaCl (1M) and 2.5 vol. of absolute ethanol (45 min at - 80°C),
6) collect the precipitate by centrifugation (45 min at 20 000g, 4°C),
7) remove the supernatant and wash the precipitated siRNA 3 times by ethanol 70% (v/v) in water (undertake centrifugation between each wash, 10 min., 2000g, 4°C, and remove the supernatant),
8) dry the precipitated siRNA at room temperature,
9) dissolve the duplex activated siRNA in phosphate buffer pH 7.1 (NaH₂PO₄/Na₂HPO₄ 50 mM, NaCl 0.5M) to a final siRNA concentration of 2 mg.mL⁻¹,
10) determine the exact siRNA concentration by UV dosage at 260 nm,
11) conjugate the activated duplex siRNA duplex with the peptide by addition of 3 eq. of CPP comprising at least one cysteine residue (10 mg.mL⁻¹ in phosphate buffer pH 7.1) followed by 1h of incubation at room temperature,
12) optionally store the reaction mixture at - 20°C,
13) optionally undertake the analysis of the reaction mixture by PAGE to evaluate conjugation efficiency,
14) precipitate the conjugate and wash as previously described (0.3 vol. of NaCl (1M) and 2.5 vol. of absolute ethanol) and air dry.

### I-2 Method for the preparation of a conjugate represented by the structure of the general formula P-L-N, wherein P is a cell penetrating peptide, N is a siRNA, and L is a polyethylene glycol (PEG)-based linker linking P and N together : double strand approach

1) dissolve hybridised siRNA (freeze-dried) with a 5'amino C3 modification for sense strand (*i.e*. NH₂-(CH₂)₃-phosphate moiety anchored on the free 5' moiety of the sense strand) in sterile water (2mM),
2) after dilution with phosphate buffer pH 8.2 (50 mM NaH₂PO₄/Na₂HPO₄ 50 mM, NaCl 0.15M) to a final siRNA concentration of 2 mg.mL⁻¹ and addition of 400 eq. of PEG-based linker (solubilized 10 mg.mL⁻¹ in N,N-diméthylformamide) incubate the reaction mixture for 90 min. at room temperature (about 20°C), followed by a 15 min incubation at 80°C,
3) allow the reaction mixture to slowly cool to room temperature to allow siRNA duplex formation,
4) precipitate the duplexes by the addition of 0.3 vol. of NaCl (1M) and 2.5 vol. of absolute ethanol (45 min at - 80°C),
5) collect the precipitate by centrifugation (45 min at 20 000g, 4°C),
6) remove the supernatant and wash the precipitated siRNA 3 times by ethanol 70% (v/v) in water (undertake centrifugation between each wash, 10 min., 2000g, 4°C, and remove the supernatant),
7) dry the precipitated siRNA at room temperature,
8) dissolve the duplex activated siRNA in phosphate buffer pH 7.1 (NaH₂PO₄/Na₂HPO₄ 50 mM, NaCl 0.5M) to a final siRNA concentration of 2 mg.mL⁻¹,
9) determine the exact siRNA concentration by UV dosage at 260 nm,
10) conjugate the activated duplex siRNA with the CPP by addition of 3 eq. of CPP comprising at least one cysteine residue (10 mg.mL⁻¹ in phosphate buffer pH 7.1) followed by 1h of incubation at room temperature,
11) optionally, store the reaction mixture at - 20°C,
12) optionally undertake the analysis of the reaction mixture by PAGE to evaluate conjugation efficiency,
13) precipitate the conjugate and wash as previously described (0.3 vol. of NaCl (1M) and 2.5 vol. of absolute ethanol) and air dry.

### I-3 Method for the preparation of a conjugate represented by the structure of the general formula P-L-N, wherein P is a cell penetrating peptide. N is an antisense nucleotide, and L is a polyethylene glycol (PEG)-based linker linking P and N together

1) dissolve the 5' amino C3 antisense oligonucleotide *(i.e.* NH₂-(CH₂)₃-phosphate moiety anchored on the free 5' moiety of the oligonucleotide) in sterile water (2mM),
2) dilute the 5' amino C3 oligonucleotide with phosphate buffer pH 8.2 (50 mM NaH₂PO₄/Na₂HPO4 50 mM, NaCl 0.15M) to a final oligonucleotide concentration of 2 mg.mL⁻¹,
3) after addition of 400 eq. of PEG-based linker (solubilized 10 mg.mL⁻¹ in N,N-dimethylformamide) incubate the reaction mixture 90 min. at room temperature (about 20 °C),
4) precipitate the oligonucleotide by the addition of 0.3 vol. of NaCl (1M) and 2.5 vol. of absolute ethanol (45 min at - 80°C),
5) collect the precipitate by centrifugation (45 min at 20 000g, 4°C),
6) remove the supernatant and wash the precipitated oligonucleotide 3 times by ethanol 70% (v/v) in water (undertake centrifugation between each wash, 10 min., 2000g, 4°C, and remove the supernatant),
7) dry the precipitated oligonucleotide at room temperature,
8) dissolve the oligonucleotide in phosphate buffer pH 7.1 (NaH₂PO₄/Na₂HPO₄ 50 mM, NaCl 0.5M) to a final siRNA concentration of 2 mg.mL⁻¹,
9) determine the exact oligonucleotide concentration by UV dosage at 260 nm,
10) conjugate the activated oligonucleotide with the peptide by addition of 3 eq. of CPP comprising at least one cysteine residue (10 mg.mL⁻¹ in phosphate buffer pH 7.1) followed by 1h of incubation at room temperature,
11) optionally store the reaction mixture at - 20°C,
12) optionally undertake the analysis of the reaction mixture by PAGE to evaluate conjugation efficiency,
13) optionally precipitate and wash the conjugate as previously described (0.3 vol. of NaCl (1M) and 2.5 vol. of absolute ethanol) and air dry.

### 1-4 Method for the preparation of the CPP-siRNA conjugates used below

The CPP-siRNA conjugates have been prepared following the method described above, paragraph I-1.

The linkers were:
- PEG-based linkers of formula III below: 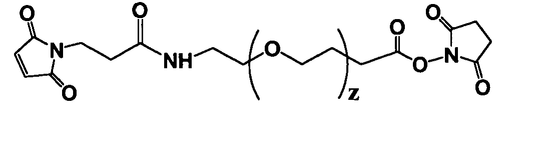
wherein k = 3 and z = 4, 12 or 80
- the sulfoSMCC linker (the PEG-based linker has been replaced by the sulfoSMCC linker in conjugation protocol) of formula VI below: 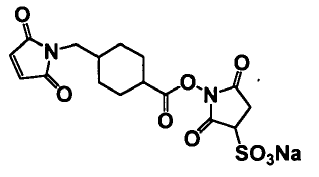 the SPDP linker (the PEG-based linker has been replaced by the SPDP linker in conjugation protocol) of formula VII below:

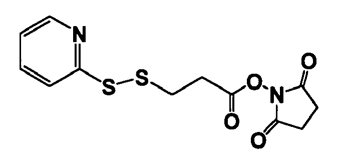

The formulas V, VIII and IX below show the linkers of formula III, VI and VII respectively once linked to a CPP containing one cysteine residue to the N- or C-terminus and a siRNA:
- formula V: 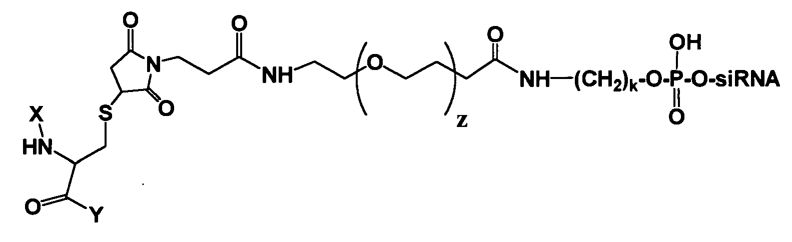
- formula VIII: 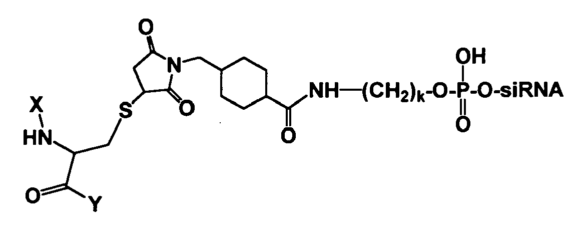
- formula IX: 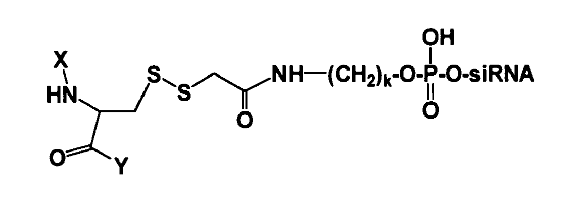
wherein:
- k = 3 ; z = 4, 12 or 80 and
- if X = H, then Y = the CPP amino acid sequence or if X = the CPP amino acid sequence, then Y = OH (depending on whether the cysteine residue is at the C- or N-terminus of the CPP)

### II- In vitro delivery of small interfering RNA for the enhanced Green Fluorescent Protein (eGFP)

The objective of the study is to compare both the internalization and efficiency intracellular delivery of active CPP-siRNA conjugates generated with different linkers (a PEG-based linker of the invention and the well known sulfoSMCC and SPDP linkers) between the CPP and the siRNA. For this purpose, the enhanced Green Fluorescent Protein (eGFP) reporter gene has been chosen.

### II-1 Materials and Methods

### II-1-1 Nucleotides sequences

siRNA were ordered from Eurogentec with the following modifications:
Sense strand: 5 prime end: amino C3
3 prime end: dTdT overhang and cy5 label (see explanation below)

Antisense strand: 3 prime end: dTdT overhang
Sequence #1, named siGFP1: 5'- GAACGGCAUCAAGGUGAAC -3' (19 nucleotides) (Ding et al., Nucleic Acids Res 32:W135-141 (2004)).
Sequence #2, named siGFP2: 5'- ACUACCAGCAGAACACCCC -3' (19 nucleotides) (Muratovska, and Eccles, FEBS Lett 558:63-68 (2004)).
Sequence #3: inactive GFP siRNA, named siGFPs: 5'- GCACGACUGGACCAAGUCC -3' (19 nucleotides) (Sorensen et al., J Mol Biol 327:761-766 (2003)).

For *in vitro* screening and activity studies, some siRNAs were stabilized by a phosphorothioate link in 3' position of the sense and antisense strands (phosphorothioate instead of phosphorate between the two thymines at the 3 prime overhang), they were named psGFP siRNA; siRNA were conjugated to CPPs with a conjugation ratio over 80%.

In order to follow the intracellular fate of the CPP-siRNA conjugate, cyanine 5 (cy5)-labeled siRNA were designed. Since cells were already fluorescein positive (because of eGFP expression), the Cy5 dye was chosen due to its wide separation of its emission from that of shorter-wavelength-emitting fluorophores. However, because of its emission maximum at 670 nm, cy5 was visualized with a confocal microscope equipped with a proper laser for excitation (*e.g*., a krypton/argon ion laser).

### II-1-2 Cell Penetrating Peptides

### DPVs:

DPV15b (23 aa: NH₂-(C)GAYDLRRRERQSRLRRRERQSR-COOH) = SEQ ID NO 12 plus one cysteine residue added to the N-terminus,

DPV3 (17 aa: NH2-RKKRRRESRKKRRRES(C)-COOH) = SEQ ID NO 2 plus one cysteine residue added to the C-terminus,

DPV1048 (19 aa: NH₂-(C)VKRGLKLRHVRPRVTRDV-COOH) = SEQ ID NO 9 plus one cysteine residue added to the N-terminus,

Penetratin long version (16 aa: NH₂-(C)RQIKIWFQNRRMKWKK-COOH) = SEQ ID NO 28 plus one cysteine residue added to the N-terminus, denoted "p16"

### II-1-3 Linkers

Stable linkers: -sulfoSMCC-,
(PEG)z-based linkers of formula III (below) wherein z = 4, 12 or 80 (named respectively -PEG₄-, -PEG₁₂- and -PEG₈₀-) 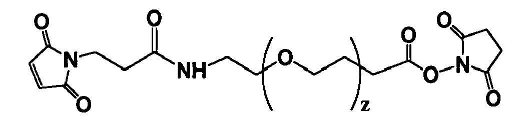
Reducible linker: -SPDP- (disulfide bond)

### II-1-4 Cell lines

GFP-EOMA: mouse endothelial cells from hemangioendothelioma stably transfected with pEGFP-N1, encoding enhanced green fluorescent protein; ATCC # CRL-2587. Cells were analyzed by Western blot due to low level of eGFP fluorescence.

Non-GFP-cell lines were included in the study to quantitate internalization with labelled siRNA:
HeLa: human epithelial cells from uterine adenocarcinoma (ATCC# CCL-2)
NCI-H460: human epithelial cells from lung carcinoma (ATCC# HTB-177)
PC-3: human epithelial cells from prostate adenocarcinoma (ATCC# CRL-1435)
MDA-MB-231: human breast cancer cells (ATCC number# HTB-26)
Cells were analyzed by flow cytometry and Western blot.

### II-1-6 Methods

### II-1-6-A eGFP transient transfected cells (HeLa, NCI-H460, PC-3 or MDA-MB-231)

Day 0: Plate cells at 1x10⁵ cells/well/ml in 24-well plates in medium with serum resulting in 90% confluence the day of transfection.

Day 1: Transient transfection with Lipofectamine^{™} 2000 (Invitrogen, ref# 11668-027) following manufacturer's instructions.

Routinely, 12.5 pmol of GFP siRNAs (siRNA control or equivalent siRNA of CPP-siRNA) +1.6 µg eGFP plasmide were resuspended following the manufacturer's protocols and diluted in 50 µL of Opti-MEM^{®} in a separate tube. Two microliters of Lipofectamine^{™} 2000 were diluted in 48 µL Opti-MEM®. The diluted siRNAs and diluted Lipofectamine^{™} 2000 were then combined, gently mixed and allowed to incubate for 30 minutes at room temperature. The siRNA/Lipofectamine^{™} 2000 mixture (100 µL) was added directly to the cells with fresh medium (400 µL). The medium was replaced with fresh medium 4 hours after transfection (1 ml) and cells were incubated 48h at 37°C prior to cell analysis.

For CPP-siRNA conjugate treatments, conjugates were directly added to the medium at the desired concentration for 48h at 37°C before harvesting.

### II-1-6-B GFP expressing cells (GFP-EOMA)

Day 0: Plate cells at 1x10⁵ cells/well/ml in 24-well plates in medium with serum.

Day 1: For DPV-siRNA treatments, conjugates were added directly to the cells in fresh medium at the desired concentration and incubate for 3 or 7 days at 37°C before harvesting as reported for Penetratin-siRNA (Muratovska, and Eccles, FEBS Lett. 558:63-68 (2004)).

### II-1-6-C Flow cytometry analysis

Cells were plated at a density of 1x10⁵ cells/well/ml in 24-well-plates and were treated as previously described. Cells were then rinsed in PBS 1X and collected using trypsine-EDTA. Cells were resuspended in ice-cold PBS-1% BSA and stored on ice until measure at the flow cytometer.

### II-1-6-D Confocal analysis

Cells were plated at a density of 1x10⁴ cells/well in glass-Labteck 8-wells (in duplicate). DPV-siRNA^{Cyanine 5} conjugates were tested at 25 or 100 nM and incubated at 37°C for few hours (24h or for the indicated time in the time-course experiment). Cells were rinsed 3 times in PBS 1X and then fixed 10 min in 4% PFA solution. Cells were finally rinsed 3 times in PBS 1X and mounted in DAPI slowfade kit before confocal observation.

### II-2 Results

### II-2-1 Cellular internalization of DPV-siRNA conjugates

Confocal microscopy clearly shows enhanced intracellular delivery of siGFP sequences in murine endothelial (GFP-EOMA) and human cancer cell lines (HeLa, PC-3, NCI-H460 and MDA-MB-231) when conjugated to DPV3, DPV15b and DPV1048 (see description above) compared to naked siRNA, using sulfoSMCC, SPDP, PEG₄-, PEG₁₂-, or PEG₈₀-based linkers. DPV-linker-siGFP conjugates are all successfully internalized and clearly located only in the cytoplasm and show a puntacte staining (endosomal/cytosolic) (data not shown).

### II-2-2 CPP-siRNA conjugate activity

The intracellular activity of internalized DPV-siRNA conjugates was first investigated in GFP-EOMA cells. While three days following treatment with DPV-linker-siGFP1 (linker = sulfoSMCC, SPDP or PEG₄-based linker) showed no inhibition of eGFP protein expression (or less than 10%), a moderate down-regulation of eGFP was observed by flow cytometry and protein expression (Western blot) seven days after treatment (up to 37%). Similar activities were obtained with DPV3 and DPV1048 siRNA conjugates (data not shown). While there was little difference between DPVs (DPV1048 is slightly less efficient *in vitro),* the PEG₄ linker clearly gave the most efficient down-regulation of eGFP expression (37% silencing effect). In addition, no clear dose-dependent response was demonstrated for DPV-Linker-siRNA in the range of concentrations tested (25, 125, 400, 500 nM), suggesting that the RNAi system may already be saturated.

A moderate down-regulation of either eGFP fluorescence or protein expression could be observed in transient transfected cells 48h after eGFP transfection followed by DPV-siRNA treatment (Table 2).

The PEG₄-based linker showed a better activity than the sulfoSMCC and the SPDP linker.

**Table 2: Silencing activity of DPV-Linker-siRNA eGFP in transient transfected PC-3 with pEGFP-N1. Analyses were performed 48 hours after transfection by flow cytometry in order to evaluate GFP fluorescence intensity. Values are compiled from "n" independent experiments.**

| | Silencing activity (% of control) | | |
|---|---|---|---|
| Linkers | DPV3 | DPV15b | DPV1048 |
| sulfoSMCC (n=7) | -5% | -7% | -5% |
| SPDP (n=6) | -5% | -7% | -4% |
| PEG₄-based linker (n=4) | 13% | -17% | -3% |
| Conclusion | DPV15b ≥ DPV3 ~ DPV 1048 | | |
| | PEG₄>> sulfoSMCC > SPDP | | |

### II-2-3 CPP-linker-siRNA conjugate efficiency

Efficiency studies comparing penetratin (long version, p16) and DPV15b linked to siRNA eGFP via a SPDP or PEG₄-based linker were performed in eGFP-transient transfected PC-3 cells. Results are shown Figure 1.

### III- In vitro evaluation of DPV 15b-SMCC-siVEGF, DPV 15b-SPDP-siVEGF, DPV 15b-PEG₄-siVEGF, DPV15b-PEG₈₀-siVEGF on VEGF secretion in MDA-MB-231 cell line

DPV15b (SEQ ID No 12) was used as a representive cell penetrating peptide for these experiments. DPV15b and siRNA specific for VEGF (Filleur et al., Cancer Res. 63:3919-22 (2003)) were conjugated using four different linkers molecules (sulfoSMCC, SPDP, a PEG₄-based linker and a PEG₈₀-based linker).

### III-1 Materials and methods

### III-1-1 Cell culture

MDA-MB-231, human breast cancer cells were obtained from American Type Culture Collection (ATCC number# HTB-26). They were cultured in Lebovitz's L-15 Medium supplemented with 10% heat inactivated FBS, glutamine (2 mM). Cells were incubated at 37 °C in 0 % CO₂ atmosphere, with 90% humidity.

### III-1-2 Chemicals

Synthesis and analyses of DPV15b-poly(ethylene glycol)₄-siVEGF (DPV15b-PEG₄-siVEGF), DPV15b-poly(ethylene glycol)₈₀-siVEGF2 (DPV15b-PEG₈₀-siVEGF), DPV15b-(N-Succinimidyl 3-[2-pyridyldithio]-propionamido)-siVEGF (DPV15b-SPDP-siVEGF) and DPV15b-Sulfosuccinimidyl-4-(N-maleimidomethyl)cyclohexane-1-carboxylate-siVEGF (DPV15b-SMCC-siVEGF) compounds have been performed as decribed above.

siVEGF sense and antisense strands were supplied by Eurogentec and hybridised by Diatos.

Sense strand: 5' AUG UGA AUG CAG ACC AAA GAA-dTsdT

### III-1-3 Transfection assays

Transfections of siRNAs and DPV-linker-siVEGF were performed either with Lipofectamine^{™} 2000 (siRNA transfection reagent, Invitrogen) to confirm that no loss of activity occurred following conjugation, or without Lipofectmine, to demonstrate DPV specific intracellular delivery. MDA-MB-231 cells (7×10⁴) were plated in 24 wells plates overnight or until they reached 70 % confluence. Transfection with Lipofectamine 2000 was undertaken using Opti-MEM I reduced serum medium (Invitrogen). Briefly, on the day of transfection, cells were washed and kept in 400 µL medium containing 10 % FBS until transfection. 25 nM of siVEGF/or DPV-linker-siVEGF and 50 µL of Opti-MEM® I medium were mixed and in a separate tube 1 µL of Lipofectamine^{™} 2000 and 50 µl of Opti-MEM® I medium were also mixed. These tubes were incubated at room temperature.

### III-1-4 VEGF ELISA assay

In order to assess VEGF secretion in the supernatants of transfected cells, media was collected after 20 hours, centrifuged to remove cellular debris, and stored at -20 °C until assayed for VEGF. VEGF was quantified using a Quantikine kit from R&D Systems according to the manufacturer's protocol. VEGF values were calculated by plotting absorbance at 450 and 540 nm and comparing the unknown values to that of the standards. VEGF expression was normalized using the total protein concentration per assay. Total cellular protein was isolated as follows: cell pellets were washed once with cold PBS and resuspended in 0.5 mL of lysis buffer (cold NaOH 1M). Protein was quantified using the Bradford assay. Data were expressed in pg VEGF/ml/mg of protein.

### III-2 Results

The siRNA activity of the DPV-siRNA conjugates was confirmed following intracellular delivery using Lipofectamine^{™} 2000. This study showed that only the conjugate with the PEG₄-based linker resulted activity equivalent to that of the naked siRNA. The sulfoSMCC and SPDP linkers showed a loss of siRNA activity compared to naked siRNA (Figure 2).

To assess the ability of DPV-siVEGF conjugates to deliver active siRNA *in vitro* the same experiment was performed but with the absence of the Lipofectamine^{™} transfection reagent. This study showed that the DPV-siVEGF molecules containing the PEG₄-based linker and PEG₈₀-based linker showed the greatest *in vitro* activity (25 % inhibition of secreted VEGF versus control was observed, Figure 2), which was greater than that observed for either the sulfoSMCC or SPDP linkers.

### IV- Evaluation of the dose-dependent efficacy of DPV15b-PEG₄-siRNA_{VEGF} conjugates in MDA-MB-231 model

DPV15b-PEG₄-psVEGF2 (named DPV15b-siRNA_{VEGF}) conjugate has been selected after *in vitro* screening. MDA-MB-231 was the cell line in which the conjugate exhibited the higher VEGF inhibition efficacy *in vitro.*

The animals were treated once a day for 5 consecutive days, by bolus i.v. injections. 25, 50 and 100 µg eq siRNA of conjugate per mouse were administered to tumor-bearing animals and compared to 50 and 100 µg naked siRNA per mouse.

### IV-1 Materials and Methods

### IV-1-1 Tumor model

MDA-MB-231 tumor cells (ATCC number: HTB-26) were grafted subcutaneously to NMRI nude mice. Treatment begun when tumors started to grow (day 6 after the graft).

### IV-1-2 Treatment

Animals were observed for 7 days in animal unit before treatment. Animal experiments were performed according to ethical guidelines of animal experimentation. The day of the first injection (6 days after the graft), mice were randomized in 7 groups. Mice were treated by intravenous injection in the lateral tail vein at a constant volume of 10 microL/g.
Groups were as follows:

| Group | Drug | Dose (µg eq siRNA/mouse) | Schedule |
|---|---|---|---|
| 1 | H₂O/NaCl | - | Q1D5 |
| 2 | psVEGF2 | 50 | Q1D5 |
| 3 | psVEGF2 | 100 | Q1D5 |
| 4 | DPV15b-PEG₄-psVEGF2 | 25 | Q1D5 |
| 5 | DPV15b-PEG₄-psVEGF2 | 50 | Q1D5 |
| 6 | DPV15b-PEG₄-psVEGF2 | 100 | Q1D5 |
| 7 | Irinotecan | 48.15 µmol/kg | Q1D5 |

### IV-1-3 Evaluation of toxicity and anti-tumoral efficacy

During the course of experiment body weight and tumor size were controlled every 2 or 3 days (except weekends). Animals were sacrificed when they showed signs of suffering, when a 30% body weight loss was observed or when their tumor volume reached approximately 10 % body weight. Mortality of animals was recorded every day except the weekend.

Tumor volume was determined twice a week from 2 dimensions caliper measurements using the formula [length x width²]/2.
The statistical analyses of the tumor growth (ANOVA1 followed by Newman-Keuls test) were realized weekly with the GraphPad prism 3.02 software.

### IV-2 Results

### IV-2-1 In vivo efficacy of DPV15b-siRNA_{VEGF} or naked siRNA_{VEGF} in MDA-MB-231 tumor model

The tumor volume evolution and statistical analyses, conducted weekly, are presented below (Figure 3)

Naked siRNA did not show any sign of activity during the course of the experiment. On the other hand, DPV15b-siRNA_{VEGF} conjugate exhibited a decrease in tumor growth beginning on day 16 (see figure 3b) and still present when control animals were sacrificed (figure 3a). This effect was dose-dependent, the maximum efficacy being observed at 100 µg, 50 µg inducing a moderate activity, 25 µg being inactive. The efficacy of DPV15b-siRNA_{VEGF} (100µg) was significant when compared to the tumor growth of animals treated with naked siRNA_{VEGF} at 100 µg on days 49, 58 and 66.

### IV-2-2 Body weight evolution, apparent toxicity

During the efficacy experiment, body weight was monitored twice weekly. Treatment toxicity was assessed based on clinical signs and body weight evolution.

No acute toxicity was observed in any group. During the course of experiment (80 days), no toxic signs (body weight loss or mortality) were reported, except for mice treated with irinotecan which showed a body weight loss of nearly 20%.

### IV-3 Conclusion

This experiment demonstrated the anti-tumoral efficacy of siRNA_{VEGF} when conjugated to DPV15b using a PEG₄-based linker. In fact, naked siRNA_{VEGF} was completely inactive at both tested doses, whereas DPV15b-siRNA_{VEGF} was active, in a dose-dependent manner, at its two higher doses, *i.e*. 50 and 100 µ eq siRNA per mouse. Moreover, no toxicity was observed with the conjugate, neither at the time of injection nor during the course of the experiment.

## Claims

1. A compound having the formula P-L-N, wherein
P is a cell penetrating peptide;
L is a hydrophilic polymer linker, preferably a PEG-based linker, linking P and N together; and
N is a nucleic acid, preferably an oligonucleotide and more preferably a siRNA.

2. The compound according to claim 1, wherein the cell penetrating peptide of said compound has the ability to translocate *in vitro* and/or *in vivo* the mammalian cell membranes and enter into cells and/or cell nuclei.

3. The compound according to claim 2, wherein P is less than or equal to 500, preferably 25 amino acids in length.

4. The compound according to claim 2, wherein P is greater than or equal to 4 amino acids in length, preferably 6 amino acids in length.

5. The compound according to claim 2, wherein P comprises an amino acids sequence selected from the group comprising SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 or SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO 49, SEQ ID NO 50.

6. The compound according to claim 2, wherein P comprises an amino-acid sequence selected from the group consisting of a) (XBBBXXBX)n ; b) (XBBXBX)n ;c) (BBXmBBXp)n ; d) (XBBXXBX)n; e) (BXBB)n or f) (an antibody fragment), wherein
each B is independently a basic amino acid preferably lysine or arginine;
each X is independently a non-basic amino acid preferably hydrophobic amino acid;
each m is independently an integer from zero to five;
each n is independently an integer between one and ten;
and each p is independently an integer between zero to five.

7. The compound according to claim 6, wherein each X is independently alanine, isoleucine, leucine, methionine, phenylalanine, tryptophan, valine or tyrosine.

8. The compound according to claim 6 or 7, wherein P comprises an amino-acid sequence of formula c).

9. The compound according to claim 8, wherein P comprises an amino acids sequence selected from the group comprising SEQ ID NO: 2, SEQ ID NO: 3, SEQ ID NO: 4, SEQ ID NO: 6, SEQ ID NO: 7.

10. The compound according to claim 6 or 7, wherein P comprises an amino-acid sequence of formula d).

11. The compound according to claim 10, wherein P is SEQ ID NO: 8, SEQ ID NO: 9, SEQ ID NO: 11.

12. The compound according to any one of the preceding claims, wherein L represents a PEG based linker, which can be obtained by the conjugation of P and N together with a group of formula II 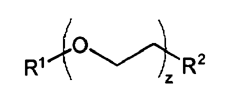 wherein
R¹ and R² are independently selected from =O, SR³, -NHR³ and -OR³, in which R³ is H, substituted or unsubstituted alkyl, substituted or unsubstituted heteroalkyl, acyl, -OR⁴, -C(O)R⁴, -C(O)OR⁴, -C(O)NR⁴R⁵, -P(O)(OR⁴)₂, -C(O)CHR⁴R⁵, -NR⁴R⁵, -N(+)R⁴R⁵R⁶, -SR⁴ or SiR⁴R⁵R⁶, and wherein R⁴, R⁵ and R⁶ independently represent H, substituted or unsubstituted alkyl, substituted or unsubstituted heteroalkyl and substituted or unsubstituted aryl, wherein R⁴ and R⁵ together with the nitrogen atom to which they are attached are optionally joined to form a substituted or unsubstituted heterocycloalkyl ring system having from 4 to 6 members, optionally containing two or more heteroatoms;
z is an integer from 1 to 10000; and
wherein R¹ has reacted with P and R² has reacted with N.

13. The compound according to claim 12, wherein z is an integer from 1 to 100, preferably from 1 to 15.

14. The compound according to claims 12 or 13, wherein L represents a PEG-based linker which can be obtained by the conjugation of P and N together with a group of formula III 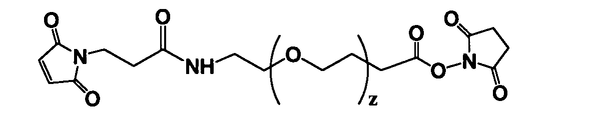 wherein z is an integer from 1 to 10000, preferably from 1 to 100.

15. The compound according to any one of the preceding claims, wherein the nucleic acid is a DNA or RNA.

16. The compound according to claim 15, wherein the RNA is a siRNA or siRNA derivative having a sequence complementary to a target mRNA sequence to direct target-specific RNA interference.

17. The compound according to claim 16, wherein the sequence identity between the siRNA or siRNA derivative and the target mRNA sequence is greater than 80%, preferably 90%.

18. The compound according to claim 16 or 17, wherein the mRNA encodes the amino acid sequence of a protein selected from the group comprising developmental proteins, oncogene-encoded proteins, tumor suppressor proteins, transcription factors, enzymes, house-keeping proteins, cytoskeleton-related proteins, receptor-related proteins, cytokines, angiogenic proteins, growth factor proteins, or pathogen-associated proteins.

19. The compound according to claim 18, wherein the mRNA encodes an amino acid sequence corresponding to an amino acid sequence of VEGF.

20. The compound according to any one of claims 16 to 19, wherein the siRNA has a length from 10 to 50 nucleotides.

21. A process for preparing a compound as defined in any of the preceding claims comprising the steps of coupling the nucleic acid with the PEG-based linker and then coupling the PEG-based linker-nucleic conjugate acid with the cell penetrating peptide.

22. Use of a PEG-based linker for conjugating a cell penetrating peptide and a nucleic acid, preferably a siRNA or siRNA derivative together.

23. Composition comprising a compound as defined in any of claims 1 to 20 and optionally a pharmaceutically acceptable carrier.

24. Use of a compound as defined in any of claims 1 to 20 for the manufacture of a medicament for treating or preventing a disorder selected from the group comprising cellular proliferative and/or differentiative disorders, disorders associated with bone metabolism, immune disorders, hematopoietic disorders, cardiovascular disorders, liver disorders, kidney disorders, muscular disorders, neurological disorders, hematological disorders, viral diseases, pain or metabolic disorders, preferably for treating cancers.


SEQUENCE LISTING

<110>  DIATOS S.A.

<120>  Cell penetrating peptide conjugates for delivering of
nucleic acids into a cell

<130>  DTS009

<160>  50

<170>  PatentIn version 3.1

<210>  1

<211>  21

<212>  PRT

<213>  Bufo gargarizans

<400>  1

Thr Arg Ser Ser Arg Ala Gly Leu Gln Phe Pro Val Gly Arg Val
1               5                   10                  15

His Arg Leu Leu Arg Lys
            20

<210>  2

<211>  16

<212>  PRT

<213>  Homo sapiens

<400>  2

Arg Lys Lys Arg Arg Arg Glu Ser Arg Lys Lys Arg Arg Arg Glu
1               5                   10                  15

Ser

<210>  3

<211>  17

<212>  PRT

<213>  Homo sapiens

<400>  3

Gly Arg Pro Arg Glu Ser Gly Lys Lys Arg Lys Arg Lys Arg Leu
1               5                   10                  15

Lys Pro

<210>  4

<211>  15

<212>  PRT

<213>  Homo sapiens

<400>  4

Gly Lys Arg Lys Lys Lys Gly Lys Leu Gly Lys Lys Arg Asp Pro
1               5                   10                  15

<210>  5

<211>  17

<212>  PRT

<213>  Homo sapiens

<400>  5

Gly Lys Arg Lys Lys Lys Gly Lys Leu Gly Lys Lys Arg Pro Arg
1               5                   10                  15

Ser Arg

<210>  6

<211>  18

<212>  PRT

<213>  Homo sapiens

<400>  6

Arg Lys Lys Arg Arg Arg Glu Ser Arg Arg Ala Arg Arg Ser Pro
1               5                   10                  15

Arg His Leu

<210>  7

<211>  19

<212>  PRT

<213>  Homo sapiens

<400>  7

Ser Arg Arg Ala Arg Arg Ser Pro Arg Glu Ser Gly Lys Lys Arg
1               5                   10                  15

Lys Arg Lys Arg

<210> 8

<211> 19

<212> PRT

<213> Homo sapiens

<400> 8

Val Lys Arg Gly Leu Lys Leu Arg His Val Arg Pro Arg Val Thr
1               5                   10                  15

Arg Met Asp Val

<210> 9

<211> 18

<212> PRT

<213> Homo sapiens

<400> 9

Val Lys Arg Gly Leu Lys Leu Arg His Val Arg Pro Arg Val Thr
1               5                   10                  15

Arg Asp Val

<210> 10

<211> 14

<212> PRT

<213> Homo sapiens

<400>  10

Ser Arg Arg Ala Arg Arg Ser Pro Arg His Leu Gly Ser Gly
1               5                   10

<210>  11

<211>  16

<212>  PRT

<213>  Homo sapiens

<400>  11

Leu Arg Arg Glu Arg Gln Ser Arg Leu Arg Arg Glu Arg Gln Ser
1               5                   10                  15

Arg

<210>  12

<211>  22

<212>  PRT

<213>  Homo sapiens

<400>  12

Gly Ala Tyr Asp Leu Arg Arg Arg Glu Arg Gln Ser Arg Leu Arg
1               5                   10                  15

Arg Arg Glu Arg Gln Ser Arg
            20

<210>  13

<211>  30

<212>  PRT

<213>  Artificial Sequence

<220>

<223>  Artificial sequence

<400>  13

Trp Glu Ala Ala Leu Ala Glu Ala Leu Ala Glu Ala Leu Ala Glu
1               5                   10                  15

His Leu Ala Glu Ala Leu Ala Glu Ala Leu Glu Ala Leu Ala Ala
            20                  25                  30

<210>  14

<211>  21

<212>  PRT

<213>  unidentified

<400>  14

Lys Gly Ser Trp Tyr Ser Met Arg Lys Met Ser Met Lys Ile Arg
1               5                   10                  15

Pro Phe Phe Pro Gln Gln
            20

<210>  15

<211>  20

<212>  PRT

<213>  unidentified

<400>  15

Lys Thr Arg Tyr Tyr Ser Met Lys Lys Thr Thr Met Lys Ile Ile
1               5                   10                  15

Pro Phe Asn Arg Leu
            20

<210>  16

<211>  20

<212>  PRT

<213>  unidentified

<400>  16

Arg Gly Ala Asp Tyr Ser Leu Arg Ala Val Arg Met Lys Ile Arg
1               5                   10                  15

Pro Leu Val Thr Gln
            20

<210>  17

<211>  24

<212>  PRT

<213>  Homo sapiens

<400>  17

Leu Gly Thr Tyr Thr Gln Asp Phe Asn Lys Phe His Thr Phe Pro
1               5                   10                  15

Gln Thr Ala Ile Gly Val Gly Ala Pro
            20

<210>  18

<211>  12

<212>  PRT

<213>  Artificial Sequence

<220>

<223>  Artificial sequence

<400>  18

Thr Ser Pro Leu Asn Ile His Asn Gly Gln Lys Leu
1               5                   10

<210>  19

<211>  12

<212>  PRT

<213>  Influenza virus

<400>  19

Asn Ser Ala Ala Phe Glu Asp Leu Arg Val Leu Ser
1               5                   10

<210>  20

<211>  30

<212>  PRT

<213>  Artificial Sequence

<220>

<223>  Artificial sequence

<400>  20

Trp Glu Ala Lys Leu Ala Lys Ala Leu Ala Lys Ala Leu Ala Lys
1               5                   10                  15

His Leu Ala Lys Ala Leu Ala Lys Ala Leu Lys Ala Cys Glu Ala
            20                  25                  30

<210>  21

<211>  16

<212>  PRT

<213>  Homo sapiens

<400>  21

Ala Ala Val Ala Leu Leu Pro Ala Val Leu Leu Ala Leu Leu Ala
1               5                   10                  15

Pro

<210>  22

<211>  10

<212>  PRT

<213>  Artificial Sequence

<220>

<223>  Artificial sequence

<400>  22

Val Pro Met Leu Lys Pro Met Leu Lys Glu
1               5                   10

<210>  23

<211>  18

<212>  PRT

<213>  Artificial Sequence

<220>

<223>  Artificial sequence

<400>  23

Lys Leu Ala Leu Lys Leu Ala Leu Lys Ala Leu Lys Ala Ala Leu
1               5                   10                  15

Lys Leu Ala

<210>  24

<211>  27

<212>  PRT

<213>  Artificial Sequence

<220>

<223>  Artificial sequence

<400>  24

Gly Ala Leu Phe Leu Gly Phe Leu Gly Ala Ala Gly Ser Thr Met
1               5                   10                  15

Gly Ala Trp Ser Gln Pro Lys Lys Lys Arg Lys Val
            20                  25

<210>  25

<211>  16

<212>  PRT

<213>  Artificial Sequence

<220>

<223>  Artificial sequence

<400>  25

Ala Ala Val Ala Leu Leu Pro Ala Val Leu Leu Ala Leu Leu Ala
1               5                   10                  15

Pro

<210>  26

<211>  9

<212>  PRT

<213>  Artificial Sequence

<220>

<223>  Artificial sequence

<400>  26

Val Gln Arg Lys Arg Gln Lys Leu Met
1               5

<210>  27

<211>  21

<212>  PRT

<213>  Artificial Sequence

<220>

<223>  Artificial sequence

<400>  27

Lys Glu Thr Trp Trp Glu Thr Trp Trp Thr Glu Trp Ser Gln Pro
1               5                   10                  15

Lys Lys Lys Arg Lys Val
            20

<210>  28

<211>  15

<212>  PRT

<213>  Artificial Sequence



<220>

<223>  Artificial sequence

<400>  28

Ser Asp Leu Trp Glu Met Met Met Val Ser Leu Ala Cys Gln Tyr
1               5                   10                  15


<210>  29

<211>  16

<212>  PRT

<213>  Drosophila antennapedia



<400>  29

Arg Gln Ile Lys Ile Trp Phe Gln Asn Arg Arg Met Lys Trp Lys
1               5                   10                  15


Lys


<210>  30

<211>  7

<212>  PRT

<213>  Drosophila antennapedia



<400>  30

Arg Arg Met Lys Trp Lys Lys
1               5

<210>  31

<211>  7

<212>  PRT

<213>  Artificial Sequence

<220>

<223>  Artificial sequence

<400>  31

Arg Arg Arg Arg Arg Arg Arg
1               5

<210>  32

<211>  9

<212>  PRT

<213>  Artificial Sequence

<220>

<223>  Artificial sequence

<400>  32

Arg Arg Arg Arg Arg Arg Arg Arg Arg
1               5

<210>  33

<211>  16

<212>  PRT

<213>  Rattus sp.

<400>  33

Arg Val Ile Arg Val Trp Phe Gln Asn Lys Arg Cys Lys Asp Lys
1               5                   10                  15

Lys

<210>  34

<211>  28

<212>  PRT

<213>  Artificial Sequence

<220>

<223>  Artificial sequence

<400>  34

Met Ala Asn Leu Gly Tyr Trp Leu Leu Ala Leu Phe Val Thr Met
1               5                   10                  15

Trp Thr Asp Val Gly Leu Cys Lys Lys Arg Pro Lys Pro
            20                  25

<210>  35

<211>  18

<212>  PRT

<213>  Mus musculus

<400>  35

Leu Leu Ile Ile Leu Arg Arg Arg Ile Arg Lys Gln Ala His Ala
1               5                   10                  15

His Ser Lys

<210>  36

<211>  18

<212>  PRT

<213>  Artificial Sequence

<220>

<223>  Artificial sequence

<400>  36

Val Arg Leu Pro Pro Pro Val Arg Leu Pro Pro Pro Val Arg Leu
1               5                   10                  15

Pro Pro Pro

<210>  37

<211>  7

<212>  PRT

<213>  Simian virus 40

<400>  37

Pro Lys Lys Lys Arg Lys Val
1               5

<210>  38

<211>  18

<212>  PRT

<213>  Porcine leukocyte

<400>  38

Arg Gly Gly Arg Leu Ser Tyr Ser Arg Arg Arg Phe Ser Thr Ser
1               5                   10                  15

Thr Gly Arg

<210>  39

<211>  10

<212>  PRT

<213>  Porcine leukocyte

<400>  39

Arg Arg Leu Ser Tyr Ser Arg Arg Arg Phe
1               5                   10

<210>  40

<211>  17

<212>  PRT

<213>  Unidentified

<400>  40

Ala Trp Ser Phe Arg Val Ser Tyr Arg Gly Ile Ser Tyr Arg Arg
1               5                   10                  15

Ser Arg

<210>  41

<211>  14

<212>  PRT

<213>  Human immunodeficiency virus type 1

<400>  41

Tyr Gly Arg Lys Lys Arg Arg Gln Arg Arg Arg Pro Pro Gln
1               5                   10

<210>  42

<211>  11

<212>  PRT

<213>  Human immunodeficiency virus type 1

<400>  42

Tyr Gly Arg Lys Lys Arg Arg Gln Arg Arg Arg
1               5                   10

<210>  43

<211>  9

<212>  PRT

<213>  Human immunodeficiency virus type 1

<400>  43

Arg Lys Lys Arg Arg Gln Arg Arg Arg
1               5

<210>  44

<211>  27

<212>  PRT

<213>  Artificial Sequence

<220>

<223>  Artificial sequence

<400>  44

Gly Trp Thr Leu Asn Ser Ala Gly Tyr Leu Leu Gly Lys Ile Asn
1               5                   10                  15

Leu Lys Ala Leu Ala Ala Leu Ala Lys Lys Ile Leu
            20                  25

<210>  45

<211>  21

<212>  PRT

<213>  Artificial Sequence

<220>

<223>  Artificial sequence

<400>  45

Ala Gly Tyr Leu Leu Gly Lys Ile Asn Leu Lys Ala Leu Ala Ala
1               5                   10                  15

Leu Ala Lys Lys Ile Leu
            20

<210>  46

<211>  12

<212>  PRT

<213>  Artificial Sequence

<220>

<223>  Artificial sequence

<400>  46

Gly Trp Thr Leu Asn Ser Ala Gly Tyr Leu Leu Gly
1               5                   10


<210>  47

<211>  14

<212>  PRT

<213>  Artificial Sequence


<220>

<223>  Artificial sequence

<400>  47

Ile Asn Leu Lys Ala Leu Ala Ala Leu Ala Lys Lys Ile Leu
1               5                   10


<210>  48

<211>  34

<212>  PRT

<213>  herpes simplex virus 1


<400>  48

Asp Ala Ala Thr Ala Thr Arg Gly Arg Ser Ala Ala Ser Arg Pro
1               5                   10                  15


Thr Glu Arg Pro Arg Ala Pro Ala Arg Ser Ala Ser Arg Pro Arg
                20                  25                  30


Arg Pro Val Asp


<210>  49

<211>  26

<212>  PRT

<213>  Artificial Sequence

<220>

<223>  Artificial sequence

<400>  49

Asp Pro Lys Gly Asp Pro Lys Gly Val Thr Val Thr Val Thr Val
1               5                   10                  15

Thr Val Thr Gly Lys Gly Asp Pro Lys Pro Asp
            20                  25

<210>  50

<211>  4

<212>  PRT

<213>  Artificial sequence

<220>

<223>  2'6'-dimethyltyrosine-D-Arg-Phe-Lys

<220>

<221>  MISC_FEATURE

<222>  (1)..(1)

<223>  Xaa= 2',6'-dimethyltyrosine

<221>  MISC_FEATURE

<222>  (2)..(2)

<223>  Arg= D-Arg

<400>  50

Xaa Arg Phe Lys
1